# EUROPEAN PATENT APPLICATION

(11) **EP 4 613 770 A1**
(43) Date of publication of application: **10.09.2025**
(21) Application number: 23886218.9
(22) Date of filing: 31.10.2023
(51) Int. Cl.: C07K 16/28, C07K 14/725, A61K 39/00, A61P 35/00

(54) **ANTIBODY BINDING TO CD22, AND BISPECIFIC ANTIBODY, CHIMERIC ANTIGEN RECEPTOR AND ANTIBODY-DRUG CONJUGATE COMPRISING SAME, AND USE THEREOF**

(30) Priority: 31.10.2022 KR 20220143216
(71) Applicant: The Asan Foundation, Seoul 05505 (KR); UNIVERSITY OF ULSAN FOUNDATION FOR INDUSTRY COOPERATION, Nam-gu Ulsan 44610 (KR)
(72) Inventor: KIM, Hyori, Seoul 05065 (KR); KOH, Kyung-Nam, Seoul 05507 (KR); KIM, Nayoung, Seoul 04969 (KR); PARK, Sunghun, Seoul 04996 (KR); KIM, Minsong, Busan 46634 (KR); HAN, Min A, Gijang-gun, Busan 46051 (KR); LEE, A-Neum, Seoul 01044 (KR)
(74) Representative: Bryn Aarflot AS
(86) International application number: PCT/KR2023/017138
(87) International publication number: WO 2024/096522

(57) **Abstract**

The present disclosure relates to an antibody binding to CD22, a bispecific antibody including the same, a chimeric antigen receptor, an antibody-drug conjugate, and uses thereof. According to one aspect, effective anticancer effects can be provided even for patients who are refractory to CD19-targeted therapies. Further, when using CD22 CAR NK cells or bispecific CD22/CD19 CAR NK cells, significantly enhanced reduction of cancer cells can be achieved.

## Description

### Technical Field

The present disclosure relates to a novel antibody binding to CD22, a bispecific antibody including the same, a chimeric antigen receptor, an antibody-drug conjugate, and uses thereof.

### Background Art

CD22 is expressed in most B-cell leukemias and lymphomas, including NHL, acute lymphoblastic leukemia (B-ALL), chronic lymphocytic leukemia (B-CLL), and especially acute non-lymphocytic leukemia (ANLL).

CD22 is detected in the cytoplasm during early B-cell development, appears on the cell surface simultaneously with IgD, and is found in most mature B cells. Its expression increases when B cells are activated, CD22 is lost at the terminal stage of differentiation, and it is generally reported to be absent in plasma cells. Furthermore, CD22 is expressed at the pre-B cell stage, maintained in mature B cells, and its expression is restricted to B cells, making it a promising target for immunotherapy of malignant B cells. Moreover, CD22-targeted therapy is considered a good alternative for CD19 downregulation or resistance to CD19-CAR therapy.

Therefore, there is a need to develop novel antibodies or fragments thereof that specifically bind to CD22 to increase the range of target diseases, and further, to develop bispecific antibodies, bispecific CARs (chimeric antigen receptors), CAR-expressing immune cells, and antibody-drug conjugates including the CD22-specific antibodies and fragments thereof.

### Disclosure of Invention

### Technical Problem

One aspect is to provide an anti-CD22 antibody or an antigen-binding fragment thereof.

Another aspect is to provide a bispecific antibody including the anti-CD22 antibody or an antigen-binding fragment thereof.

Another aspect is to provide a nucleic acid encoding the antibody or an antigen-binding fragment thereof.

Another aspect is to provide a host cell expressing the nucleic acid.

Another aspect is to provide a chimeric antigen receptor (CAR) including the antibody or an antigen-binding fragment thereof.

Another aspect is to provide an immune cell expressing the chimeric antigen receptor.

Another aspect is to provide an antibody-drug conjugate (ADC) including the anti-CD22 antibody or an antigen-binding fragment thereof.

Another aspect is to provide a pharmaceutical composition for preventing or treating a B cell-mediated disease, the composition including the antibody, the bispecific antibody, or the antibody-drug conjugate.

### Solution to Problem

One aspect provides an antibody or fragment thereof that specifically binds to CD22.

In the present specification, the terms "antibody," "antigen-binding region or site," or "antigen-binding polypeptide" may refer to a polypeptide or a polypeptide complex that specifically recognizes an antigen and binds thereto. The antibody may be a whole antibody, an antigen-binding fragment thereof, or a single chain thereof. Accordingly, the term "antibody" may include any protein or peptide-containing molecule that may include at least a portion of an immunoglobulin molecule having the biological activity of binding to an antigen. Such examples include, but are not limited to, a complementarity determining region (CDR) of a heavy chain or light chain or a ligand-binding portion thereof, a heavy chain or light chain variable region, a heavy chain or light chain constant region, a framework (FR) region, or any portion thereof, or at least one portion of a binding protein.

In an embodiment, the antibody includes, but is not limited to, a monoclonal antibody, monospecific antibody, bispecific antibody, trispecific antibody, diabody, multispecific antibody, multivalent antibody, minibody, domain antibody, antibody mimetic (or synthetic antibody), chimeric antibody, humanized antibody, or antibody fusion (or antibody conjugate), and fragments thereof, and may include various forms of antibodies disclosed herein.

In the present specification, the terms "antigen" or "immunogen" may mean a molecule or a portion of a molecule to which, for example, an antigen-binding protein (e.g., an antibody or an immunologically functional antigen-binding fragment thereof) may bind, and which may be used for the generation of antibodies that may bind to the antigen in an animal. An antigen may comprise one or more epitopes that may interact with different antibodies or fragments thereof.

In the present specification, the terms "antibody fragment" or "antigen-binding fragment" may include a portion of an antibody that lacks some amino acids compared to a full-length chain but may specifically bind to an antigen. Such a fragment may be considered to have biological activity in that it may specifically bind to a target antigen or may compete with another antibody or antigen-binding fragment for binding to a specific epitope. In one aspect, such a fragment may include at least one CDR present in a full-length light chain or heavy chain, and, in some embodiments, it may comprise a truncated heavy chain and/or light chain, or a portion thereof. Such a biologically active fragment may be produced by recombinant DNA technology, or, for example, may be produced by enzymatic or chemical cleavage of an intact antibody. Immunologically functional immunoglobulin fragments include, but are not limited to, Fab, Fab', F(ab')2, scFab, dsFv, Fv, scFv, scFv-Fc, scFab-Fc, diabodies, minibodies, scAb, dAb, half-IgG, or combinations thereof. Furthermore, it may be derived from any mammal including, but not limited to, a human, mouse, rat, camelid, or rabbit. A functional portion of an antibody, such as one or more CDRs described herein, may be linked by a covalent bond to a secondary protein or a low molecular weight compound, and thereby may be used as a targeted therapeutic agent for a specific target. The term "antibody fragment" includes aptamers, spiegelmers, and diabodies. The term "antibody fragment" may also include any synthetic or genetically engineered protein that acts like an antibody by binding to a specific antigen and forming a complex.

The "Fc" region may comprise two heavy chain fragments including the CH2 and CH3 domains of an antibody. These two heavy chain fragments may be combined with each other by two or more disulfide bonds and hydrophobic interactions of the CH3 domains.

The "Fab fragment" may consist of one heavy chain including only the CH1 and variable regions and one light chain. The heavy chain of an Fab molecule may not form a disulfide bond with another heavy chain molecule.

The "Fab' fragment" includes the components of a Fab fragment with an additional region from the heavy chain between the CH1 and CH2 domains, which may form disulfide bonds between the heavy chains of two Fab' fragment molecules to form an F(ab')2 molecule.

The "F(ab')2 fragment," as described above, includes two heavy chains that include a variable region, CH1, and a portion of the constant region between the CH1 and CH2 domains, and two light chains, whereby interchain disulfide bonds are formed between the two heavy chains. Accordingly, an F(ab')2 fragment consists of two Fab' fragments, and the two Fab' fragments may be linked to each other by disulfide bonds between them.

The "Fv region" may be an antibody that includes respective variable regions of the heavy chain and the light chain, but does not include a constant region. An scFv may be an Fv in which these variable regions are connected by a flexible linker. An scFv-Fc may be an scFv linked to an Fc. A minibody may be an scFv linked to a CH3 domain. A diabody may include two molecules of scFv. A "single-chain variable fragment" or "scFv" may refer to a fusion protein of the variable regions of the heavy chain (VH) and the light chain (VL) of an immunoglobulin. In some aspects, these regions may be connected by a short linker peptide of 10 to about 25 amino acids. The linker may be rich in glycine for flexibility and serine or threonine for solubility, and may link the N-terminus of VH with the C-terminus of VL, or vice versa. Such a protein may retain the specificity of the original immunoglobulin, despite the removal of constant regions and the introduction of the linker. ScFv molecules are known in the art and, for example, are described in U.S. Patent No. 5,892,019.

In the present specification, a "short-chain antibody (scAb)" may be a single polypeptide chain including one variable region of a heavy or light chain constant region, wherein the heavy and light chain variable regions are linked by a flexible linker. Short-chain antibodies are, for example, described in U.S. Patent No. 5,260,203.

In the present specification, a "domain antibody (dAb)" may be an immunologically functional immunoglobulin fragment including only the variable region of a heavy chain or the variable region of a light chain. In an embodiment, two or more VH regions may be covalently linked by a peptide linker to form a bivalent domain antibody. The two VH regions of such a bivalent domain antibody may target the same or different antigens.

In the present specification, a "bivalent antigen-binding protein" or "bivalent antibody" may comprise two antigen-binding sites. The two antigen-binding sites comprised in such a bivalent antibody may have the same antigen specificity, or the bivalent antibody may be a bispecific antibody wherein the two antigen-binding sites bind separately to different antigens.

According to an embodiment, the CD22 antibody or an antigen-binding fragment thereof may be an antibody or fragment thereof that specifically binds to CD22 and may include: a light chain variable region including: a CDR1 region represented by the amino acid sequence of SEQ ID NO: 1 or 2, a CDR2 region represented by the amino acid sequence of SEQ ID NO: 3 or 4, and a CDR3 region represented by the amino acid sequence of SEQ ID NO: 5 or 6; and a heavy chain variable region including: a CDR1 region represented by an amino acid sequence selected from SEQ ID NOS: 7 to 12, a CDR2 region represented by an amino acid sequence selected from SEQ ID NOS: 13 to 15, and a CDR3 region represented by an amino acid sequence selected from SEQ ID NOS: 16 to 19.

**[Table 1]**

| Name | Sequence | SEQ ID |
|---|---|---|
| VL CDR1 | SGGSGGYG | SEQ ID NO: 1 |
| | SGSNNTYG | SEQ ID NO: 2 |
| VL CDR2 | DNDQRPS | SEQ ID NO: 3 |
| | DNTNRPS | SEQ ID NO: 4 |
| VL CDR3 | GSYDSSYVGI | SEQ ID NO: 5 |
| | GSRDSTYVGI | SEQ ID NO: 6 |
| VH CDR1 | GFTFSNYCMQ | SEQ ID NO: 7 |
| | GFTFSNYCMS | SEQ ID NO: 8 |
| | GFTFSKHGHY | SEQ ID NO: 9 |
| | GFTFSRHGMY | SEQ ID NO: 10 |
| | GFSFSDYGMG | SEQ ID NO: 11 |
| | GFAFGRSSMG | SEQ ID NO: 12 |
| VH CDR2 | GIGSSGTGTYYGSAVRG | SEQ ID NO: 13 |
| | SISNTGSYTDYGAAVKG | SEQ ID NO: 14 |
| | FLGSSSSYTDYGAAVKG | SEQ ID NO: 15 |
| VH CDR3 | DYGSDSGCGWGIYAGEIDA | SEQ ID NO: 16 |
| | DYGSDSGIGWGIYAGEIDA | SEQ ID NO: 17 |
| | DYGSDSGVGWGIYAGEIDA | SEQ ID NO: 18 |
| | SAYGGSYGSSSIDA | SEQ ID NO: 19 |

In an embodiment, the CDRs of the light chain variable region and the CDRs of the heavy chain variable region disclosed in Table 1 may be freely combined.

In an embodiment, the CD22 antibody or an antigen-binding fragment thereof may comprise a light chain variable region represented by an amino acid sequence selected from the group consisting of SEQ ID NOS: 20 to 22. In an embodiment, the CD22 antibody or an antigen-binding fragment thereof may comprise a heavy chain variable region represented by an amino acid sequence selected from the group consisting of SEQ ID NOS: 23 to 29. In an embodiment, the CD22 antibody or an antigen-binding fragment thereof may comprise: a light chain variable region represented by an amino acid sequence selected from the group consisting of SEQ ID NOS: 20 to 22; and a heavy chain variable region represented by an amino acid sequence selected from the group consisting of SEQ ID NOS: 23 to 29.

**[Table 2]**

| Nam e | Sequence | SEQ ID |
|---|---|---|
| VL | LTQPSSVSANPGETVKITCSGGSGGYGWYQQKSPGSAPVSLIYDN DQRPSDIPSRFSGSKSG STGTLTITGVQADDEAVYYCGSYDSSYVGIFGAGTTLTVL | SEQ ID NO: 20 |
| | LTQPPSVSVSPGQTARITCSGGSGGYGWYQQKPGSAPVSVIYDND QRPSGIPERFSGSSSGTTVTLTISGVQAEDEADYYCGSYDSSYVGIF GGGTKLTVL | SEQ ID NO: 21 |
| | LTQPSSVSANPGETVKITCSGSNNTYGWYQQKAPGSAPVTVIYDNT NRPSDIPSRFSGSKSGSTNTLTITGVQAEDEAVYFCGSRDSTYVGIF GAGTTLTVL | SEQ ID NO: 22 |
| VH | AVTLDESGGGLQTPGGALSLVCKASGFTFSNYCMQWVRQAPGKGL EWVAGIGSSGTGTYYGS AVRGRATISRDNGQSTVRLQLNNLRAEDTGTYYCARDYGSDSGCG WGIYAGEIDAWGHGTEV IVSS | SEQ ID NO: 23 |
| | EVQLLESGGGLVQPGGSLRLSCAASGFTFSNYCMSWVRQAPGKGL EWVSGIGSSGTGTYYGSAVRGRFTISRDNSKNTLYLQMNSLRAEDT AVYYCARDYGSDSGCGWGIYAGEIDA WGQGTLVTVSS | SEQ ID NO: 24 |
| | EVQLLESGGGLVQPGGSLRLSCAASGFTFSKHGHYWVRQAPGKGL EWVSGIGSSGTGTYYGSAVRGRFTISRDNSKNTLYLQMNSLRAEDT AVYYCARDYGSDSGIGWGIYAGEIDA WGQGTLVTVSS | SEQ ID NO: 25 |
| | EVQLLESGGGLVQPGGSLRLSCAASGFTFSRHGMYWVRQAPGKG LEWVSGIGSSGTGTYYGSAVRGRFTISRDNSKNTLYLQMNSLRAED TAVYYCARDYGSDSGVGWGIYAGEIDA WGQGTLVTVSS | SEQ ID NO: 26 |
| | AVTLDESGGGLQTPGGGLSLVCKASGFSFSDYGMGWVRQAPGKG LEFVASISNTGSYTDYGAAVKGRATISRDNGQSTLRLQLNNLRAEDT GTYFCAKSAYGGSYGSSSIDAWGHGTEVIVSS | SEQ ID NO: 27 |
| | AVTLDESGGGLQTPGGGLSLVCKASGFAFGRSSMGWVRQAPGKG LEFVASISNTGSYTDYGAAVKGRATISRDNGQSTLRLQLNNLRAEDT GTYFCAKSAYGGSYGSSSIDA WGHGTEVIVSS | SEQ ID NO: 28 |
| | AVTLDESGGGLQTPGGGLSLVCKASGFSFSDYGMGWVRQAPGKG LEFVAFLGSSSSYTDYGAAVKGRATISRDNGQSTLRLQLNNLRAEDT GTYFCAKSAYGGSYGSSSIDA WGHGTEVIVSS | SEQ ID NO: 29 |

In an embodiment, the light chain variable region and the heavy chain variable region disclosed in Table 2 may be freely combined. The antibody and/or an antigen-binding fragment thereof may include "variants". The variants may refer to polypeptides in which one or more amino acid residues from the polypeptide sequence are inserted, deleted, added, and/or substituted. Therefore, as long as the desired biological activity and/or structure of the antibody and/or antigen-binding fragment is maintained, the variants may include fusion polypeptides formed by connecting to other polypeptides. The variant may have a sequence identity to the sequence of the antibody or an antigen-binding fragment thereof of at least 80 %, at least 85 %, at least 90 %, at least 95 %, at least 96 %, at least 97 %, at least 98 %, or at least 99 %, and, for example, may have a sequence identity thereto of about 99 %, 98 %, 97 %, 96 %, 95 %, 94 %, 93 %, 92 %, 91 %, 90 %, 89 %, 88 %, 87 %, 86 %, 85 %, 84 %, 83 %, 82 %, 81 %, or 80 %.

According to an embodiment, the variant may have at least 80 %, at least 85 %, at least 90 %, at least 95 %, at least 96 %, at least 97 %, at least 98 %, or at least 99 % sequence identity to the sequence of an antibody that specifically binds to CD22 or fragment thereof, including: a light chain variable region including a CDR1 region represented by the amino acid sequence of SEQ ID NO: 1 or 2, a CDR2 region represented by the amino acid sequence of SEQ ID NO: 3 or 4, and a CDR3 region represented by the amino acid sequence of SEQ ID NO: 5 or 6; and a heavy chain variable region including a CDR1 region represented by an amino acid sequence selected from SEQ ID NOS: 7 to 12, a CDR2 region represented by an amino acid sequence selected from SEQ ID NOS: 13 to 15, and a CDR3 region represented by an amino acid sequence selected from SEQ ID NOS: 16 to 19. For example, the variant may have a sequence identity of about 99 %, 98 %, 97 %, 96 %, 95 %, 94 %, 93 %, 92 %, 91 %, 90 %, 89 %, 88 %, 87 %, 86 %, 85 %, 84 %, 83 %, 82 %, 81 %, or 80 %.

According to an embodiment, the variant may have at least 80 %, at least 85 %, at least 90 %, at least 95 %, at least 96 %, at least 97 %, at least 98 %, or at least 99 % sequence identity to the sequence of an antibody that specifically binds to CD22 or fragment thereof, including: a light chain variable region represented by the amino acid sequence of any one selected from the group consisting of SEQ ID NOS: 20 to 22; and a heavy chain variable region represented by the amino acid sequence of any one selected from the group consisting of SEQ ID NOS: 23 to 29. For example, the variant may have a sequence identity of about 99 %, 98 %, 97 %, 96 %, 95 %, 94 %, 93 %, 92 %, 91 %, 90 %, 89 %, 88 %, 87 %, 86 %, 85 %, 84 %, 83 %, 82 %, 81 %, or 80 %.

In the present specification, the term "identity" may refer to the overall relatedness between nucleic acid molecules and/or between polypeptides. In an embodiment, nucleic acids or polypeptides may be considered "substantially identical" to each other if their sequences are at least 25 %, 30 %, 35 %, 40 %, 45 %, 50 %, 55 %, 60 %, 65 %, 70 %, 75 %, 80 %, 85 %, 90 %, 95 %, 96 %, 97 %, 98 %, or 99 % identical. The calculation of percent identity between two nucleic acid or polypeptide sequences may be performed, for example, by aligning the two sequences for optimal comparison purposes. In certain embodiments, the length of the sequence aligned for comparison purposes may be at least 30 %, at least 40 %, at least 50 %, at least 60 %, at least 70 %, at least 75 %, at least 80 %, at least 85 %, at least 90 %, at least 95 %, at least 96 %, at least 97 %, at least 98 %, at least 99 %, or substantially 100 % of the length of the reference sequence.

The anti-CD22 antibody or fragment thereof described in the present specification may be, without limitation, a chimeric antibody, a humanized antibody, or a fully human antibody. In one aspect, the antibody or fragment thereof does not occur naturally and may be synthesized chemically or by recombination. Additionally, the CD22 antibody or antigen-binding fragment thereof in the present specification may include amino acid sequences engineered from the amino acid sequences of SEQ ID NOS: 1 to 29.

In the present specification, the term "humanized antibody" may refer to an antibody possessing amino acid sequences corresponding to those of antibodies produced by humans and/or an antibody made using techniques for creating human antibodies.

In an embodiment, the antibody specifically binding to CD22 or fragment thereof may further include a human constant kappa region (Cκ) and a Hemagglutinin (HA) tag protein. In an embodiment, the humanized antibody or antigen-binding fragment thereof may be represented by the amino acid sequence of SEQ ID NO: 30 or 31.

In an embodiment, the humanized antibody or antigen-binding fragment thereof may further comprise human immunoglobulin heavy chain and light chain regions. For example, the human immunoglobulin may be an IgG.

In an embodiment, the humanized antibody or antigen-binding fragment thereof may be represented by an amino acid sequence selected from the group consisting of SEQ ID NOS: 32 to 43.

Another aspect provides a bispecific antibody including a CD22 antibody or an antigen-binding fragment thereof.

In the present specification, the term "bispecific", "dual-specific" antigen-binding protein or antibody may be a hybrid antigen-binding protein or antibody having two different antigen-binding sites. Such a bispecific antibody is a type of multispecific antigen-binding protein or multispecific antibody, which may be produced by various known methods such as linking of Fab' fragments or fusion of hybridomas. For example, reference may be made to Songsivilai and Lachmann, 1990, Clin. Exp. Immunol. 79:315-321; Kostelny et al., 1992, J. Immunol. 148:1547-1553, and the like. The two different epitopes to which the two antigen-binding sites of a bispecific antigen-binding protein or antibody bind may be located on the same or different protein targets.

According to an embodiment, the bispecific antibody may be an anti-CD22 and anti-cotinine bispecific antibody.

The anti-CD22 and anti-cotinine bispecific antibody may comprise an anti-CD22 antibody or an antigen-binding fragment thereof as a CD22-targeting moiety capable of specifically recognizing and/or binding to a CD22 protein, and may comprise an anti-cotinine antibody or an antigen-binding fragment thereof as a cotinine-targeting moiety capable of specifically recognizing and/or binding to cotinine.

In the present specification, the term "cotinine" refers to a major metabolite of nicotine and has the structure of Formula 1 below.

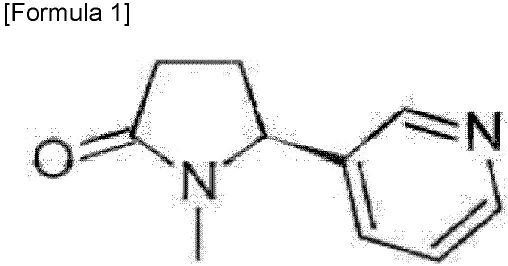

In an embodiment, the anti-CD22 and anti-cotinine bispecific antibody may include an anti-CD22 antibody or fragment thereof; and an anti-cotinine antibody or fragment thereof, wherein the anti-CD22 antibody or fragment thereof may comprise: a light chain variable region including a CDR1 region represented by the amino acid sequence of SEQ ID NO: 1 or 2, a CDR2 region represented by the amino acid sequence of SEQ ID NO: 3 or 4, and a CDR3 region represented by the amino acid sequence of SEQ ID NO: 5 or 6; and a heavy chain variable region including a CDR1 region represented by an amino acid sequence selected from SEQ ID NOS: 7 to 12, a CDR2 region represented by an amino acid sequence selected from SEQ ID NOS: 13 to 15, and a CDR3 region represented by an amino acid sequence selected from SEQ ID NOS: 16 to 19.

In an embodiment, the anti-CD22 and anti-cotinine bispecific antibody may include an anti-CD22 antibody or fragment thereof and an anti-cotinine antibody or fragment thereof, wherein the anti-CD22 antibody or fragment thereof may include a light chain variable region represented by an amino acid sequence selected from the group consisting of SEQ ID NOS: 20 to 22.

In an embodiment, the anti-CD22 and anti-cotinine bispecific antibody may include an anti-CD22 antibody or fragment thereof and an anti-cotinine antibody or fragment thereof, wherein the anti-CD22 antibody or fragment thereof may include a heavy chain variable region represented by an amino acid sequence selected from the group consisting of SEQ ID NOS: 23 to 29.

In an embodiment, the anti-CD22 and anti-cotinine bispecific antibody may include an anti-CD22 antibody or fragment thereof and an anti-cotinine antibody or fragment thereof, wherein the anti-CD22 antibody or fragment thereof may include: a light chain variable region represented by an amino acid sequence selected from the group consisting of SEQ ID NOS: 20 to 22; and a heavy chain variable region represented by an amino acid sequence selected from the group consisting of SEQ ID NOS: 23 to 29.

In an embodiment, the anti-CD22 and anti-cotinine bispecific antibody may include an anti-CD22 antibody or fragment thereof and an anti-cotinine antibody or fragment thereof, wherein the anti-CD22 antibody or fragment thereof is humanized. Specifically, the anti-CD22 and anti-cotinine bispecific antibody may include an anti-CD22 antibody or fragment thereof and an anti-cotinine antibody or fragment thereof, wherein the anti-CD22 antibody or fragment thereof further may include a human constant kappa region (Cκ) and an HA tag (Hemagglutinin tag) protein, or further include human immunoglobulin heavy chain and light chain regions. More specifically, the anti-CD22 and anti-cotinine bispecific antibody may include an anti-CD22 antibody or fragment thereof and an anti-cotinine antibody or fragment thereof, wherein the anti-CD22 antibody is represented by the amino acid sequence of SEQ ID NO: 44 or 46.

The human constant kappa region (Cκ), the HA tag (Hemagglutinin tag) protein, and the human immunoglobulin heavy chain and light chain regions are as described above.

According to an embodiment, the bispecific antibody may be an anti-CD22 and anti-CD3 bispecific antibody.

The anti-CD22 and anti-CD3 bispecific antibody may comprise an anti-CD22 antibody or an antigen-binding fragment thereof as a CD22-targeting moiety capable of specifically recognizing and/or binding to a CD22 protein, and may comprise an anti-CD3 antibody or an antigen-binding fragment thereof as a CD3-targeting moiety capable of specifically recognizing and/or binding to a CD3 protein. In an embodiment, the anti-CD22 and anti-CD3 bispecific antibody may include an anti-CD22 antibody or fragment thereof and an anti-CD3 antibody or fragment thereof, wherein the anti-CD22 antibody or fragment thereof may include: a light chain variable region including: a CDR1 region represented by the amino acid sequence of SEQ ID NO: 1 or 2, a CDR2 region represented by the amino acid sequence of SEQ ID NO: 3 or 4, and a CDR3 region represented by the amino acid sequence of SEQ ID NO: 5 or 6; and a heavy chain variable region including: a CDR1 region represented by an amino acid sequence selected from SEQ ID NOS: 7 to 12, a CDR2 region represented by an amino acid sequence selected from SEQ ID NOS: 13 to 15, and a CDR3 region represented by an amino acid sequence selected from SEQ ID NOS: 16 to 19.

In an embodiment, the anti-CD22 and anti-CD3 bispecific antibody may include an anti-CD22 antibody or fragment thereof and an anti-CD3 antibody or fragment thereof, wherein the anti-CD22 antibody or fragment thereof may include a light chain variable region represented by an amino acid sequence selected from the group consisting of SEQ ID NOS: 20 to 22.

In an embodiment, the anti-CD22 and anti-CD3 bispecific antibody may include an anti-CD22 antibody or fragment thereof and an anti-CD3 antibody or fragment thereof, wherein the anti-CD22 antibody or fragment thereof may include a heavy chain variable region represented by an amino acid sequence selected from the group consisting of SEQ ID NOS: 23 to 29.

In an embodiment, the anti-CD22 and anti-CD3 bispecific antibody may include an anti-CD22 antibody or fragment thereof and an anti-CD3 antibody or fragment thereof, wherein the anti-CD22 antibody or fragment thereof may include: a light chain variable region represented by an amino acid sequence selected from the group consisting of SEQ ID NOS: 20 to 22; and a heavy chain variable region represented by an amino acid sequence selected from the group consisting of SEQ ID NOS: 23 to 29.

In an embodiment, the anti-CD22 and anti-CD3 bispecific antibody may include an anti-CD22 antibody or fragment thereof and an anti-CD3 antibody or fragment thereof, wherein the anti-CD22 antibody or fragment thereof is humanized. Specifically, the anti-CD22 and anti-CD3 bispecific antibody may include an anti-CD22 antibody or fragment thereof and an anti-CD3 antibody or fragment thereof, wherein the anti-CD22 antibody or fragment thereof further may include a human constant kappa region (Cκ) and an HA tag (Hemagglutinin tag) protein, or further includes human immunoglobulin heavy chain and light chain regions. More specifically, the anti-CD22 and anti-CD3 bispecific antibody may be represented by an amino acid sequence selected from the group consisting of SEQ ID NOS: 47 to 54.

The human constant kappa region (Cκ), the HA tag (Hemagglutinin tag) protein, and the human immunoglobulin heavy chain and light chain regions are as described above.

According to an embodiment, the bispecific antibody may be an anti-CD22 and anti-CD19 bispecific antibody.

The anti-CD22 and anti-CD19 bispecific antibody may include an anti-CD22 antibody or antigen-binding fragment thereof as a CD22-targeting moiety capable of specifically recognizing and/or binding to a CD22 protein, and may include an anti-CD19 antibody or antigen-binding fragment thereof as a CD19-targeting moiety capable of specifically recognizing and/or binding to a CD19 protein. In an embodiment, the anti-CD22 and anti-CD19 bispecific antibody may include an anti-CD22 antibody or fragment thereof and an anti-CD19 antibody or fragment thereof, wherein the anti-CD22 antibody or fragment thereof may include: a light chain variable region including: a CDR1 region represented by the amino acid sequence of SEQ ID NO: 1 or 2, a CDR2 region represented by the amino acid sequence of SEQ ID NO: 3 or 4, and a CDR3 region represented by the amino acid sequence of SEQ ID NO: 5 or 6; and a heavy chain variable region including: a CDR1 region represented by an amino acid sequence selected from SEQ ID NOS: 7 to 12, a CDR2 region represented by an amino acid sequence selected from SEQ ID NOS: 13 to 15, and a CDR3 region represented by an amino acid sequence selected from SEQ ID NOS: 16 to 19.

In an embodiment, the anti-CD22 and anti-CD19 bispecific antibody may include an anti-CD22 antibody or fragment thereof and an anti-CD19 antibody or fragment thereof, wherein the anti-CD22 antibody or fragment thereof may include a light chain variable region represented by an amino acid sequence selected from the group consisting of SEQ ID NOS: 20 to 22.

In an embodiment, the anti-CD22 and anti-CD19 bispecific antibody may include an anti-CD22 antibody or fragment thereof and an anti-CD19 antibody or fragment thereof, wherein the anti-CD22 antibody or fragment thereof may include a heavy chain variable region represented by an amino acid sequence selected from the group consisting of SEQ ID NOS: 23 to 29.

In an embodiment, the anti-CD22 and anti-CD19 bispecific antibody may include an anti-CD22 antibody or fragment thereof and an anti-CD19 antibody or fragment thereof, wherein the anti-CD22 antibody or fragment thereof may include: a light chain variable region represented by an amino acid sequence selected from the group consisting of SEQ ID NOS: 20 to 22; and a heavy chain variable region represented by an amino acid sequence selected from the group consisting of SEQ ID NOS: 23 to 29.

In an embodiment, the anti-CD22 and anti-CD19 bispecific antibody may include an anti-CD22 antibody or fragment thereof and an anti-CD19 antibody or fragment thereof, wherein the anti-CD22 antibody or fragment thereof is humanized. Specifically, the anti-CD22 and anti-CD19 bispecific antibody may include an anti-CD22 antibody or fragment thereof and an anti-CD19 antibody or fragment thereof, wherein the anti-CD22 antibody or fragment thereof further includes a human constant kappa region (Cκ) and an HA tag (Hemagglutinin tag) protein, or further includes human immunoglobulin heavy chain and light chain regions.

The human constant kappa region (Cκ), the HA tag (Hemagglutinin tag) protein, and the human immunoglobulin heavy chain and light chain regions are as described above.

Another aspect provides a nucleic acid encoding the antibody or antigen-binding fragment thereof.

In the present specification, the term "nucleic acid" or "polynucleotide" may include single-stranded and double-stranded nucleotide polymers. The nucleotides constituting a polynucleotide may be ribonucleotides or deoxyribonucleotides, or modified forms of either type of nucleotide. The modifications may include base modifications such as bromouridine and inosine derivatives, ribose modifications such as 2',3'-dideoxyribose, and internucleotide linkage modifications such as phosphorothioate, phosphorodithioate, phosphoroselenoate, phosphorodiselenoate, phosphoroanilothioate, phosphoraniladate, and phosphoramidate.

The nucleic acid sequence encoding the antibody and/or antigen-binding fragment thereof may be cloned into various types of vectors. For example, the nucleic acid may be cloned into plasmids, phagemids, phage derivatives, animal viruses, and cosmids. Other vectors may include expression vectors, replication vectors, probe generation vectors, sequencing vectors, and viral vectors. In another example, the vector may be a foamy viral (FV) vector, which is a type of retroviral vector derived from spumaviruses. The design and techniques of viral vectors are widely known in the art, as described in Sambrook et al. (Molecular Cloning: A Laboratory Manual, 2001) and other virology and molecular biology manuals.

In the present specification, the term "vector" may refer to a nucleic acid molecule that is introduced into a host cell to produce a transformed host cell. The vector may comprise a nucleic acid sequence, such as an origin of replication, that allows replication in a host cell. A vector may also comprise one or more therapeutic genes and/or selectable marker genes and other genetic elements known in the art. A vector may transduce, transform, or infect a cell to cause the cell to express nucleic acids and/or proteins other than those native to the cell. A vector may optionally comprise materials that facilitate the entry of the nucleic acid into cells, such as viral particles, liposomes, protein coatings, etc.

Another aspect provides a host cell that is transformed with the vector and expresses the nucleic acid.

In the present specification, the term "host cell" may refer to a cell which has been transformed, or is capable of being transformed, with a nucleic acid sequence to express a gene of interest. A host cell may include progeny of the parent cell, as long as the gene of interest is present, regardless of whether the progeny are identical to the original parent cell in morphology or genetic makeup.

Another aspect provides a nucleic acid encoding the bispecific antibody, a vector including the nucleic acid, and a cell transformed with the vector. The "nucleic acid," "vector," and "cell" are as described above.

Another aspect provides a chimeric antigen receptor (CAR) including the antibody or antigen-binding fragment thereof.

In the present specification, the term "chimeric antigen receptor" or "CAR" may refer to an artificially constructed hybrid protein or polypeptide containing an antigen-binding domain of an antibody (e.g., a single-chain variable fragment (scFv)) linked to a domain or signaling domain, for example, a T-cell signaling or T-cell activation domain that activates an immune cell (e.g., a T cell or an NK cell). A CAR may utilize the antigen-binding properties of a monoclonal antibody to redirect immune cell specificity and reactivity to a selected target in an MHC-unrestricted manner.

In an embodiment, the chimeric antigen receptor may comprise an antigen-binding domain, a transmembrane domain, a hinge domain, an intracellular co-stimulatory domain, and an intracellular signal transduction domain.

In the present specification, the terms "binding domain," "extracellular domain," "extracellular binding domain," "antigen-specific binding domain," and "extracellular antigen-specific binding domain" may be used interchangeably, and may refer to a domain or fragment of a chimeric antigen receptor (CAR) having the ability to specifically bind to a target antigen (e.g., CD22).

In an embodiment, the antigen-binding domain of the chimeric antigen receptor may be linked to a transmembrane domain by a hinge domain.

In the present specification, the term "transmembrane domain" generally refers to a portion that passes through the cell membrane, is connected to an intracellular signal transduction domain, and plays a role in signal transduction. The transmembrane domain may be derived from a protein selected from the group consisting of CD8α, CD4, CD28, CD137, CD80, CD86, CD152, and PD1, and preferably, the transmembrane domain of the chimeric antigen receptor may be a transmembrane domain of CD8.

In the present specification, the term "hinge domain" means a domain consisting of any oligopeptide or polypeptide, may comprise 1 to 100 amino acid residues, preferably 10 to 70 amino acid residues, and may comprise all or part of a CD8-derived hinge domain, but is not limited thereto.

In the present specification, the term "co-stimulatory domain" generally refers to an intracellular domain capable of providing an immune-stimulating molecule, which is a cell surface molecule required for an effective response of lymphocytes to an antigen. The co-stimulatory domain may include an intracellular co-stimulatory domain of CD28, and may include an intracellular co-stimulatory domain from the TNF receptor family, such as OX40 and 4-1BB.

In the present specification, the term "intracellular signal transduction domain" generally refers to a domain that is located inside a cell and is capable of transducing signals. In the present disclosure, the intracellular signal transduction domain is an intracellular signal transduction domain of a chimeric antigen receptor. For example, the intracellular signal transduction domain may be selected from the group consisting of intracellular signal transduction domains of CD3 zeta (ζ), CD3 gamma (γ), CD3 delta (δ), CD3 epsilon (ε), FcR gamma, FcR beta, CD5, CD22, CD79a, CD79b, and CD66d, but is not limited thereto.

In an embodiment, the chimeric antigen receptor may further comprise a Myc tag and/or a signal peptide at the N-terminus of the antigen-binding domain.

In the present specification, the term "antigen-binding domain of a chimeric antigen receptor" means the portion in a chimeric antigen receptor that functions to recognize and bind to a specific antigen.

In an embodiment, the antigen-binding domain of the chimeric antigen receptor may comprise a CD19 antibody or antigen-binding fragment thereof. For example, the CD19 binding domain may utilize FMC63. Specifically, the chimeric antigen receptor including the CD19 antibody or an antigen-binding fragment thereof (CD19 CAR) may be represented by the amino acid sequence of SEQ ID NO: 49.

In an embodiment, the antigen-binding domain of the chimeric antigen receptor may comprise a CD22 antibody or antigen-binding fragment thereof.

Specifically, the antigen-binding domain of the chimeric antigen receptor (CD22 CAR) including the CD22 antibody or antigen-binding fragment thereof may comprise: a light chain variable region including a CDR1 region represented by the amino acid sequence of SEQ ID NO: 1 or 2, a CDR2 region represented by the amino acid sequence of SEQ ID NO: 3 or 4, and a CDR3 region represented by the amino acid sequence of SEQ ID NO: 5 or 6; and a heavy chain variable region including a CDR1 region represented by an amino acid sequence selected from SEQ ID NOS: 7 to 12, a CDR2 region represented by an amino acid sequence selected from SEQ ID NOS: 13 to 15, and a CDR3 region represented by an amino acid sequence selected from SEQ ID NOS: 16 to 19.

Specifically, the antigen-binding domain of the chimeric antigen receptor (CD22 CAR) including the CD22 antibody or antigen-binding fragment thereof may comprise: a light chain variable region represented by an amino acid sequence selected from the group consisting of SEQ ID NOS: 20 to 22; and a heavy chain variable region represented by an amino acid sequence selected from the group consisting of SEQ ID NOS: 23 to 29.

More specifically, the chimeric antigen receptor (CD22 CAR) including the CD22 antibody or antigen-binding fragment thereof may be represented by the amino acid sequence of SEQ ID NO: 56.

Another aspect provides a bispecific chimeric antigen receptor (CD19/CD22 bispecific CAR).

In the present specification, the term "bispecific chimeric antigen receptor" refers to a chimeric antigen receptor that may simultaneously recognize and target two or more different antigens, may include two or more different antigen-binding domains, and these antigen-binding domains may specifically bind to different antigens.

Specifically, the bispecific chimeric antigen receptor may comprise an antigen-binding domain, a transmembrane domain, a hinge domain, an intracellular co-stimulatory domain, and an intracellular signal transduction domain. The bispecific chimeric antigen receptor may include two or more antigen-binding domains that recognize different antigens, and these antigen-binding domains that recognize different antigens may be connected by a linker.

In an embodiment, the bispecific chimeric antigen receptor may be a CD19/CD22 bispecific chimeric antigen receptor (CD19/CD22 bispecific CAR). Specifically, the CD19/CD22 bispecific chimeric antigen receptor may comprise a CD22 antibody or antigen-binding fragment thereof and a CD19 binding domain.

In the present specification, the term "CD19 binding domain" refers to an antigen-binding domain that specifically binds to CD19, meaning a CD19 antibody or antigen-binding fragment thereof. In an embodiment, in a CD19/CD22 bispecific chimeric antigen receptor (CD19/CD22 bispecific CAR), the CD19 binding domain may be connected by a linker to an antigen-binding domain that specifically binds to CD22.

In an embodiment, the CD19/CD22 bispecific chimeric antigen receptor may have the C-terminus of the CD19 binding domain linked to the N-terminus of the CD22 binding domain by a linker.

In an embodiment, the CD19/CD22 bispecific chimeric antigen receptor may have the C-terminus of the CD22 binding domain linked to the N-terminus of the CD19 binding domain by a linker.

In an embodiment, the CD19/CD22 bispecific chimeric antigen receptor may comprise components sequentially connected in the order of: a light chain variable region of an antibody that specifically binds to CD19, a heavy chain variable region of an antibody that specifically binds to CD22, a light chain variable region of an antibody that specifically binds to CD22, and a heavy chain variable region of an antibody that specifically binds to CD19.

Specifically, the CD19/CD22 chimeric antigen receptor may be represented by an amino acid sequence selected from the group consisting of SEQ ID NOS: 57 to 59.

Another aspect provides a nucleic acid encoding the chimeric antigen receptor or bispecific chimeric antigen receptor, a vector including the nucleic acid, and a cell expressing the nucleic acid.

In an embodiment, the cell expressing the nucleic acid encoding the chimeric antigen receptor or the bispecific chimeric antigen receptor may be an immune cell. More specifically, the immune cell may refer to a cell that regulates immunity by resisting pathogens or toxins that have invaded a living body, and may include natural killer cells, T cells, T lymphocytes, B cells, dendritic cells, or macrophages. Additionally, the immune cell may be an autologous or allogeneic immune cell.

In the present specification, the term "Natural Killer cells" or "NK cells" refers to cytotoxic lymphocytes that constitute a major component of the innate immune system, are defined as large granular lymphocytes (LGLs), and may constitute a third cell lineage differentiated from B and T lymphocytes generated from common lymphoid progenitors (CLPs). The "Natural Killer cells" or "NK cells" include natural killer cells derived from any tissue source without additional modification, and may include natural killer precursor cells as well as mature natural killer cells. The natural killer cells are activated in response to interferons or macrophage-derived cytokines, and natural killer cells may comprise two types of surface receptors that control the cytotoxic activity of the cell, termed "activating receptors" and "inhibitory receptors". Natural killer cells may be generated from hematopoietic cells, for example, hematopoietic stem or progenitor cells, from any source such as placental tissue, placental perfusate, umbilical cord blood, placental blood, peripheral blood, spleen, liver, etc.

The natural killer cells may be genetically modified or engineered. In the present specification, "genetic modification" or "genetic engineering" may include artificially altering the composition or structure of the genetic material of a cell.

In an embodiment, a genetically modified natural killer cell may be a natural killer cell including a chimeric antigen receptor. A chimeric antigen receptor may be an artificial membrane-bound protein that directs an immune cell (e.g., a T lymphocyte) against an antigen and stimulates the immune cell to kill a cell expressing the antigen. A chimeric antigen receptor includes an extracellular domain that binds to an antigen (e.g., an antigen on a cell), a transmembrane domain, an intracellular (cytoplasmic) signaling domain (i.e., an intracellular stimulating domain) that transmits a primary activation signal to an immune cell, and/or a co-stimulatory domain. When all other conditions are met, and when a chimeric antigen receptor is expressed on the surface of, for example, a T lymphocyte (e.g., a primary T lymphocyte), and the intracellular domain of the chimeric antigen receptor binds to an antigen, the intracellular signaling domain transmits a signal to the T lymphocyte to activate and/or proliferate it, and also may kill the cell expressing the antigen if the antigen is present on the cell surface. Some immune cells, for example, T lymphocytes and natural killer cells, require two signals for maximal activation, namely, a primary activation signal and a co-stimulatory signal, and a CAR may also include a co-stimulatory domain such that binding of an antigen to the extracellular domain causes the transmission of both a primary activation signal and a co-stimulatory signal.

Another aspect provides an antibody-drug conjugate (ADC) including a CD22 antibody or antigen-binding fragment thereof.

Another aspect provides an antibody-drug conjugate (ADC) including a bispecific antibody that includes a CD22 antibody or antigen-binding fragment thereof.

Specifically, the antibody-drug conjugate may comprise: a CD22 antibody or antigen-binding fragment thereof, or a bispecific antibody including the CD22 antibody or antigen-binding fragment thereof, or an antigen-binding fragment of the bispecific antibody; a drug moiety; and a linker connecting the drug moiety and the antibody or antigen-binding fragment thereof.

In an embodiment, the antibody-drug conjugate including a bispecific antibody that includes the CD22 antibody or antigen-binding fragment thereof may be an antibody-drug conjugate including an anti-CD22 and anti-cotinine bispecific antibody.

In the present specification, the term "antibody-drug conjugate (ADC)" may refer to a substance in which a cytotoxic small molecule drug (payload) is conjugated to an antibody or antigen-binding fragment thereof that binds to a specific target antigen on a cell surface.

In an embodiment, the antibody-drug conjugate may comprise a therapeutic agent (drug) conjugated via a linker to an antibody or an antigen-binding fragment thereof having target specificity.

The linker may include a cleavable linker or a non-cleavable linker. The cleavable linker, such as a peptide linker, may be cleaved by intracellular peptidases or protease enzymes, for example, lysosomal or endosomal proteases. For example, the cleavable linker may be selected from the group consisting of a protease cleavable linker, an acid-cleavable linker, a disulfide linker, a β-glucuronide-based linker, and a β-galactoside-based linker, but is not limited thereto. With the non-cleavable linker, a drug may be released after the antibody is non-selectively degraded by intracellular hydrolysis.

According to an embodiment, the therapeutic agent may be selected from the group consisting of a chemotherapeutic compound, a cytotoxic compound, an immunomodulatory compound, an anticancer agent, an antiviral agent, an antibacterial agent, an antifungal agent, an antiparasitic agent, and combinations thereof. For example, the cytotoxic compound may be selected from the group consisting of a tubulin inhibitor (mitotic inhibitor), a DNA alkylating agent, a topoisomerase inhibitor, and combinations thereof, but is not limited thereto. More specifically, the therapeutic agent may be MMAE_Protein A, cotinine-duocarmycin, or Herceptin, but is not limited thereto.

Another aspect provides a pharmaceutical composition for treating cancer, including the antibody, the antigen-binding fragment, the bispecific antibody, or the antibody-drug conjugate.

In the present specification, the term "cancer" or tumor may collectively refer to a disease caused by cells having an aggressive characteristic wherein cells ignore normal growth limits and divide and proliferate, an invasive characteristic of infiltrating surrounding tissues, and a metastatic characteristic of spreading to other parts of the body.

The cancer may be a hematologic cancer or a solid tumor.

The hematologic cancer may be selected from the group consisting of acute myeloid leukemia, acute lymphoblastic leukemia, chronic myelogenous leukemia, multiple myeloma, and lymphoma, but is not limited thereto.

The solid cancer may be selected from the group consisting of breast cancer, colorectal cancer, head and neck cancer, lung cancer, stomach cancer, skin cancer, colon cancer, prostate cancer, bladder cancer, kidney cancer, rectal cancer, thyroid cancer, liver cancer, cervical cancer, skin cancer, rectal cancer, anal cancer, urethral cancer, ovarian cancer, esophageal cancer, and pancreatic cancer, but is not limited thereto.

Another aspect provides a pharmaceutical composition for preventing or treating a B cell-mediated disease, including the antibody, the antigen-binding fragment, the bispecific antibody, or the antibody-drug conjugate.

In the present specification, the term "B cell-mediated disease" may refer to a disease mediated by abnormal activation or function of B cells. Specifically, the B cell-mediated disease may be selected from the group consisting of tumor, lymphoma, non-Hodgkin's lymphoma (NHL) aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma, but is not limited thereto.

The pharmaceutical composition may contain the antibody or antigen-binding fragment thereof as an active ingredient in an amount of about 0.1 % by weight to about 90 % by weight, specifically about 0.5 % by weight to about 75 % by weight, and more specifically about 1 % by weight to about 50 % by weight, based on the total weight of the composition.

The pharmaceutical composition may comprise conventional, non-toxic, pharmaceutically acceptable additives that are formulated into a preparation according to conventional methods. For example, the pharmaceutical composition may further comprise a pharmaceutically acceptable carrier, diluent, or excipient.

Examples of additives used in the pharmaceutical composition may include sweetening agents, binders, solvents, solubilizing agents, wetting agents, emulsifying agents, isotonic agents, absorbents, disintegrants, antioxidants, preservatives, lubricants, glidants, fillers, flavoring agents, and the like. For example, the additives may include lactose, dextrose, sucrose, mannitol, sorbitol, cellulose, glycine, silica, talc, stearic acid, stearin, magnesium stearate, magnesium aluminosilicate, starch, gelatin, tragacanth gum, alginic acid, sodium alginate, methylcellulose, sodium carboxymethylcellulose, agar, water, ethanol, polyethylene glycol, polyvinylpyrrolidone, sodium chloride, calcium chloride, orange essence, strawberry essence, vanilla flavor, and the like.

The pharmaceutical composition may be formulated into various dosage forms for oral administration (for example, tablets, pills, powders, capsules, syrups, or emulsions) or parenteral administration (for example, intramuscular, intravenous, or subcutaneous injection).

Specifically, the pharmaceutical composition may be formulated as a preparation for oral administration, and additives used therein may include cellulose, calcium silicate, corn starch, lactose, sucrose, dextrose, calcium phosphate, stearic acid, magnesium stearate, calcium stearate, gelatin, talc, surfactants, suspending agents, emulsifying agents, diluents, and the like. Furthermore, examples of glidants include colloidal silicon dioxide, magnesium silicate, and the like; examples of diluents include microcrystalline cellulose, lactose anhydrous, lactose monohydrate, silicified MCC HD 90, and the like; examples of disintegrants include croscarmellose sodium, crospovidone, and the like; and examples of lubricants include magnesium stearate, sodium lauryl sulfate, stearic acid, and the like.

Furthermore, liquid preparations for oral administration may include suspensions, emulsions, syrups, and the like, and in addition to commonly used simple diluents such as water and liquid paraffin, various excipients, for example, wetting agents, sweetening agents, flavoring agents, preservatives, and the like may be included.

Furthermore, preparations for parenteral administration include sterile aqueous solutions, non-aqueous solvents, suspensions, emulsions, lyophilized preparations, and suppositories. As non-aqueous solvents and suspending agents, propylene glycol, polyethylene glycol, vegetable oils such as olive oil, injectable esters such as ethyl oleate, and the like may be used. As suppository bases, Witepsol, macrogol, Tween 61, cacao butter, laurin butter, glycerogelatin, and the like may be used. Meanwhile, injectables may include conventional additives such as solubilizers, isotonic agents, suspending agents, emulsifiers, stabilizers, preservatives, and the like.

The pharmaceutical composition may be administered to a patient in a therapeutically effective amount or a pharmaceutically effective amount.

The term "therapeutically effective amount" or "pharmaceutically effective amount" refers to an amount of a compound or composition effective to prevent or treat a target disease, which is an amount sufficient to treat the disease at a reasonable benefit/risk ratio applicable to medical treatment and is not enough to cause side effects. The level of the effective amount may be determined according to factors including the patient's health status, type of disease, severity, activity of the drug, sensitivity to the drug, method of administration, time of administration, route of administration and excretion rate, duration of treatment, drugs used in combination or concurrently, and other factors well known in the medical field.

The pharmaceutical composition may be administered as an individual therapeutic agent or in combination with other therapeutic agents, may be administered sequentially or simultaneously with conventional therapeutic agents, and may be administered in single or multiple doses. Considering all the aforementioned factors, it is important to administer an amount that yields the maximum effect with a minimal amount without side effects, and this may be readily determined by those skilled in the art.

Specifically, the effective amount of the antibody or antigen-binding fragment thereof in the pharmaceutical composition may vary depending on the patient's age, sex, and body weight, and generally, about 0.1 mg to about 1,000 mg per kg of body weight, or about 5 mg to about 200 mg per kg of body weight, may be administered daily or every other day, or may be administered in one to three divided doses per day. However, since the dosage may be increased or decreased according to the administration route, severity of the disease, sex, body weight, age, and the like, the range is not limited thereto.

Furthermore, the pharmaceutical composition may be administered for tumor therapy in combination with chemotherapy, radiation therapy, immunotherapy, hormone therapy, bone marrow transplantation, stem cell replacement therapy, other biological therapies, surgical intervention, or a combination thereof. For example, the pharmaceutical composition may be used as an adjuvant therapy in conjunction with other long-term treatment strategies, or it may be used to maintain the patient's condition after tumor regression or chemopreventive therapy in severely ill patients.

### Advantageous Effects of Invention

According to one aspect, an effective cancer treatment effect can be exhibited even in patients refractory to CD19 therapeutics. Furthermore, when CD22 CAR NK cells or bispecific CD22/CD19 CAR NK cells are used, a significantly enhanced effect in reducing the number of cancer cells is observed.

### Description of Drawings

FIG. 1 is a graph showing results of an enzyme immunoassay for the recombinant anti-CD22 scFv-Cκ-HA fusion protein.
FIG. 2 is a graph showing flow cytometry analysis for anti-CD22 scFv-Cκ-HA fusion protein 2-3-14 and CI4-47.
FIG. 3 is a graph showing enzyme immunoassay results for the anti-CD22 recombinant protein.
FIG. 3A is a graph showing enzyme immunoassay results for the anti-CD22 IgG (C14-47_8-1, CI4-47_8-1_H1-2-1, CI4-47_8-1_H1-2-37) protein, and
FIG. 3B is a graph showing enzyme immunoassay results for the anti-CD22 IgG (2-3-14, 2-3-14_H1-24, 2-3-14_H2-40) protein.
FIG. 4 is a graph showing flow cytometry analysis of the anti-CD22 recombinant protein.
FIG. 4A is a graph showing flow cytometry analysis for the anti-CD22 IgG proteins CI4-47_8-1, CI4-47_8-1_H1-2-1, and CI4-47_8-1_H1-2-37, and FIG. 4B is a graph showing flow cytometry analysis for the anti-CD22 IgG proteins 2-3-14, 2-3-14_H1-24, and 2-3-14_H2-40.
FIG. 5 is a schematic diagram showing structures of anti-CD19 and anti-CD22 single-specific and bispecific CARs.
FIG. 5A is a schematic diagram showing the anti-CD19 and anti-CD22 single-specific CAR structure, and FIG. 5B is a schematic diagram showing the anti-CD19/CD22 bispecific CAR structure.
FIG. 6 is a schematic diagram showing an in vitro evaluation method for anti-CD19, anti-CD22, and anti-CD19/CD22 bispecific CAR-NK-92 cells.
FIG. 7 is a graph showing AV/7-AAD measured by flow cytometry after co-culturing CAR-NK-92 cells and CFSE-labeled OCI-Ly7 cells.
FIG. 8A is a graph showing AV+ 7-AAD- early apoptotic cells and AV+ 7-AAD+ late apoptotic cells (%) among CFSE+ target cells, and FIG. 8B is a graph showing numbers of CFSE+ target cells.
FIG. 9 is a graph showing in vivo cancer cell removal ability of anti-CD19/CD22 bispecific CAR-NK-92 cells.
FIG. 9A is a schematic diagram of an in vivo experimental evaluation method, and FIG. 9B is a graph showing in vivo cancer cell removal ability of anti-CD19/CD22 bispecific CAR-NK-92 cells.
FIG. 10 is a graph showing in vitro cytotoxicity results of Anti-CD22 x anti-cotinine bispecific antibody and cotinine-duocarmycin.
FIG. 11 is a graph showing in vitro cytotoxicity results of the Anti-CD22 IgG and MMAE_Protein A complex.
FIG. 11A shows cytotoxicity test results for the anti-CD22 (CI4-47_8-1) IgG and MMAE_Protein A complex, and FIG. 11B shows cytotoxicity test results for the anti-CD22 (2-3-14, 2-3-14_H1-24, 2-3-14_H2-40) IgG and MMAE_Protein A complex.
FIG. 12 is a graph showing in vitro IL-2 secretion assay results for the Anti-CD22 x anti-CD3 bispecific antibody.

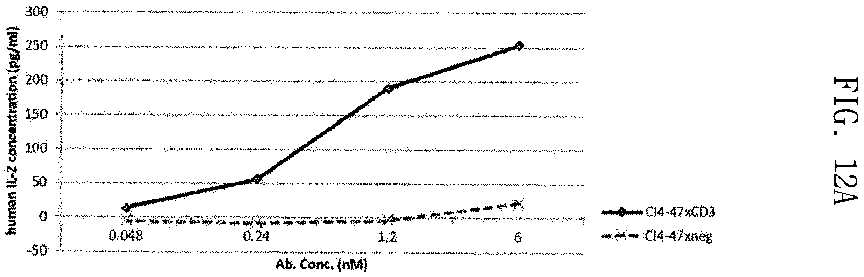

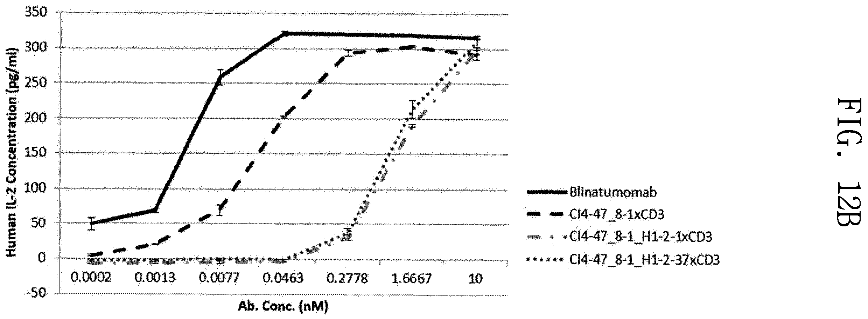

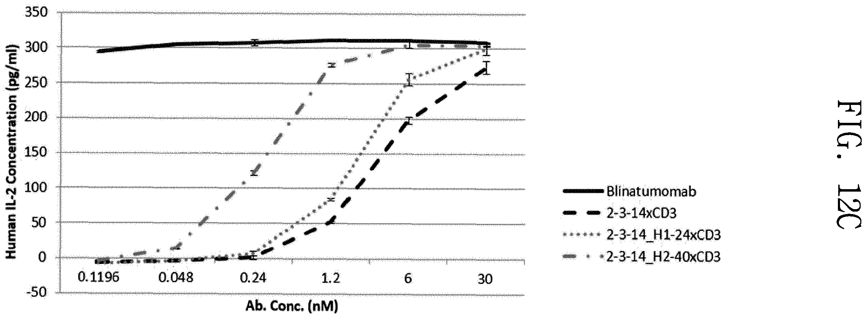

FIG. 12A shows IL-2 secretion assay results of the C14-47 x anti-CD3 (blinatumomab) bispecific antibody treatment group and the C14-47 x irrelevant antibody negative control antibody treatment group. FIG. 12B shows IL-2 secretion assay results of blinatumomab, CI4-47_8-1 x anti-CD3 (blinatumomab), CI4-47_8-1_H1-2-1 x anti-CD3 (blinatumomab), and CI4-47_8-1_H1-2-37 x anti-CD3 (blinatumomab) bispecific antibody treatment groups. FIG. 12C shows IL-2 secretion assay results of blinatumomab, 2-3-14 x anti-CD3 (blinatumomab), 2-3-14_H1-24 x anti-CD3 (blinatumomab), and 2-3-14_H2-40 x anti-CD3 (blinatumomab) bispecific antibody treatment groups.

### Mode for Invention

The present disclosure will now be described in greater detail through the following examples. However, these examples are provided for illustrative purposes only, and the scope of the present disclosure is not limited thereto.

### Reference Example 1. Cell Culture

NK-92 cells were purchased from American Type Culture Collection (Manassas, VA, USA) and maintained in NK92 medium, in α-MEM: NK92 medium is α-MEM (Welgene, Gyeongsangbuk-do, Republic of Korea) medium containing 12.5 % non-heat inactivated fetal bovine serum (FBS) (Welgene), 12.5 % heat-inactivated horse serum (Gibco, Thermo Fisher Scientific, Waltham, MA, USA), 10 U/ml penicillin, 10 µg/ml streptomycin (Welgene), 20 mM Folic acid (Sigma-Aldrich, USA), 20 mM Myo-inositol (Sigma-Aldrich), 55 mM 2-mercaptoethanol (Thermo Fisher Scientific) and 100 U/ml recombinant human interleukin (rhIL)-2 (PeproTech, Rocky Hill, NH, USA).

Human B cell lymphoma cell line OCI-Ly7 (Korean Cell Line Bank, Seoul, Korea) and HEK293T cells (Korean Cell Line Bank, Seoul, Korea) were maintained in DMEM (Welgene) medium containing 10 % heat-inactivated FBS (Welgene), 100 U/ml penicillin, and 100 µg/ml streptomycin (Welgene).

### Reference Example 2. Generation of CAR Transduced Cells and Flow Cytometry Analysis

Plasmid DNA was transformed by heat shock into competent *E*. *coli* (Stbl3) prepared using CaCl₂ 0.1 M solution according to standard protocols. The CAR vector was isolated using a Plasmid DNA Midi preparation kit (Qiagen, Venlo, Netherlands).

To produce lentiviral particles, the CAR-expression vector was transfected into HEK293T cells together with the packaging plasmid vectors pCAG-KGP1-1R, pCAG4-RTR2, and pCAG-VSVG at a ratio of 6:3:1:1 using Lipofectamine 3000 (Invitrogen, Waltham, MA, USA). The virus-containing medium was harvested 48 hours and 72 hours post-transfection and filtered through a 0.45 µM filter (Millipore, Burlington, MA, USA). Expanded NK92 cells, pre-treated with 8 µg/ml polybrene (Millipore, Burlington, Massachusetts, USA), were transduced using the viral supernatant. For CAR-NK-92 cells, NK-92 cells were sorted by a BD FACSAria^{™}II cell sorter to isolate approximately 80-99 % CAR-expressing (Myc+) cells.

Flow cytometry analysis was performed using an APC CD56 (NCAM) monoclonal antibody (mAb) (Clone CMSSB; Invitrogen), AlexaFluor-647-conjugated Myc-Tag mouse mAb (Clone 9B11; Cell Signaling Technology, Beverly, MA, USA), or a matched AlexaFluor-647-conjugated mouse IgG2a, κ isotype control (Clone MOPC-173; BioLegend). CAR-NK92 cells were cryopreserved in heat-inactivated FBS (Welgene) containing 10 % dimethyl sulfoxide (Sigma-Aldrich) and stored in a liquid nitrogen tank until use.

### Reference Example 3. Cytotoxicity Assay

Cytotoxicity was analyzed using a 7-aminoactinomycin D (7-AAD) and Annexin V (AV) assay. Target cells, OCI-Ly7 cells, were labeled with CFSE (carboxyfluorescein diacetate succinimidyl ester) using a CellTrace Cell Proliferation Kit (Thermo Fisher Scientific) according to the manufacturer's protocol. On day 2 post-transduction, CAR-αβ T and γδ T cells were harvested and resuspended in complete RPMI medium, and CAR-NK92 cells were resuspended in NK92 medium. CAR-transduced cells (effector cells) were co-cultured with CFSE-labeled target cells at an effector:target ratio of 2:1. After incubating overnight, the cells were washed with Annexin V binding buffer (BD Biosciences) and incubated for 30 minutes at 37 °C. In the case of CAR-NK92 cells, the incubation was for 4 hours. The cells were treated with human FcR blocking reagent (Miltenyi Biotec, Bergisch Gladbach, Germany) for 5 minutes and then stained on ice for 15 minutes with 7-AAD Viability Staining Solution (BioLegend) and Pacific Blue^{™} Annexin V antibody (BioLegend). CFSE-positive target cells were evaluated for early and late apoptosis by flow cytometry using a CytoFLEX (Beckman Coulter Life Sciences). Data analysis was performed using FlowJo v10 (Treestar, Inc., Ashland, OR, USA).

### Reference Example 4. In Vivo Xenograft Tumor Model Study in NOD/SCID/γc-/-(NSG) Mice

NSG (NOD.Cg-PrkdcscidIl2rgtm1Wjl/SzJ) mice (JABio, Suwon, Republic of Korea) were purchased, and OCI-Ly7 cells were labeled with CFSE. On Day 0, 5 x 10⁶ cells/200 µl in 1x PBS (BioSsesang, Republic of Korea) were injected intraperitoneally into NSG mice. After 30 minutes, 5 x 10⁶ CAR-transduced cells were injected intraperitoneally into the mice. On Day 2, 15 ml of 1x PBS was injected into the peritoneal cavity. Subsequently, peritoneal lavage fluid was harvested from the mice, and the percentage of CFSE+ cell and the number of CFSE+ cells were measured by flow cytometry. Data were analyzed using FlowJo v10 software. All mouse experimental procedures were performed with the approval of the Animal Care Committee of Seoul Asan Hospital Animal Hospital (Approval No. 2022-12-090).

### Reference Example 5. Statistical Analysis

Graphical and statistical analyses were performed using GraphPad Prism v6 software (GraphPad Software Inc., San Diego, CA, USA). p-values were calculated by one-way analysis of variance (ANOVA) with multiple comparisons. P<0.05 was considered statistically significant. All data are presented as mean ± standard error of the mean (SEM).

### Example 1. Generation of Anti-CD22 scFv Antibodies

Three White Leghorn chickens were immunized with 20 µg of recombinant human CD22-Fc protein (Cat.No. 1968-SL, R&D Systems, MN, USA) and boosted 3 times. Spleen, bursa of Fabricius, and bone marrow were harvested, and total RNA was isolated using TRI Reagent (Invitrogen, CA, USA). To generate an scFv display phage library, complementary DNA was synthesized using the Superscript IV First-Strand Synthesis system (Invitrogen, CA, USA) (C.F. Barbas, Phage Display: A Laboratory Manual, Cold Spring Harbor Laboratory Press, Cold Spring Harbor, New York, 2001).

The library was subjected to 5 rounds of biopanning using CD22-binding magnetic beads (catalog number 14302, Invitrogen). To select for binders, the reactivity of antibody-displaying phages was tested in a phage enzyme immunoassay using phage-containing culture supernatants and a horseradish peroxidase (HRP)-conjugated anti-M13 antibody (GE Healthcare, NJ, USA). Phage clones exhibiting a positive signal (OD₄₀₅ ≥ 0.6) were selected, and their nucleotide sequences were determined. Examples of the anti-CD22 antibody or antigen-binding fragments thereof obtained by this method are described in Table 3 and Table 4.

**[Table 3]**

| Name | Sequence | SEQ ID |
|---|---|---|
| VL CDR1 | SGGSGGYG | SEQ ID NO: 1 |
| | SGSNNTYG | SEQ ID NO: 2 |
| VL CDR2 | DNDQRPS | SEQ ID NO: 3 |
| | DNTNRPS | SEQ ID NO: 4 |
| VL CDR3 | GSYDSSYVGI | SEQ ID NO: 5 |
| | GSRDSTYVGI | SEQ ID NO: 6 |
| VH CDR1 | GFTFSNYCMQ | SEQ ID NO: 7 |
| | GFTFSNYCMS | SEQ ID NO: 8 |
| | GFTFSKHGHY | SEQ ID NO: 9 |
| | GFTFSRHGMY | SEQ ID NO: 10 |
| | GFSFSDYGMG | SEQ ID NO: 11 |
| | GFAFGRSSMG | SEQ ID NO: 12 |
| VH CDR2 | GIGSSGTGTYYGSAVRG | SEQ ID NO: 13 |
| | SISNTGSYTDYGAAVKG | SEQ ID NO: 14 |
| | FLGSSSSYTDYGAAVKG | SEQ ID NO: 15 |
| VH CDR3 | DYGSDSGCGWGIYAGEIDA | SEQ ID NO: 16 |
| | DYGSDSGIGWGIYAGEIDA | SEQ ID NO: 17 |
| | DYGSDSGVGWGIYAGEIDA | SEQ ID NO: 18 |
| | SAYGGSYGSSSIDA | SEQ ID NO: 19 |

**[Table 4]**

| Name | Sequence | SEQ ID |
|---|---|---|
| VL | LTQPSSVSANPGETVKITCSGGSGGYGWYQQKSPGSA PVSLIYDNDQRPSDIPSRFSGSKSG STGTLTITGVQADDEAVYYCGSYDSSYVGIFGAGTTLTV L | SEQ ID NO: 20 |
| | LTQPPSVSVSPGQTARITCSGGSGGYGWYQQKPGSAP VSVIYDNDQRPSGIPERFSGSSSGTTVTLTISGVQAEDE ADYYCGSYDSSYVGIFGGGTKLTVL | SEQ ID NO: 21 |
| | LTQPSSVSANPGETVKITCSGSNNTYGWYQQKAPGSA PVTVIYDNTNRPSDIPSRFSGSKSGSTNTLTITGVQAED EAVYFCGSRDSTYVGIFGAGTTLTVL | SEQ ID NO: 22 |
| VH | AVTLDESGGGLQTPGGALSLVCKASGFTFSNYCMQWV RQAPGKGLEWVAGIGSSGTGTYYGS AVRGRATISRDNGQSTVRLQLNNLRAEDTGTYYCARDY GSDSGCGWGIYAGEIDAWGHGTEV IVSS | SEQ ID NO: 23 |
| | EVQLLESGGGLVQPGGSLRLSCAASGFTFSNYCMSWV RQAPGKGLEWVSGIGSSGTGTYYGSAVRGRFTISRDN SKNTLYLQMNSLRAEDTAVYYCARDYGSDSGCGWGIYA GEIDA WGQGTLVTVSS | SEQ ID NO: 24 |
| | EVQLLESGGGLVQPGGSLRLSCAASGFTFSKHGHYWV RQAPGKGLEWVSGIGSSGTGTYYGSAVRGRFTISRDN SKNTLYLQMNSLRAEDTAVYYCARDYGSDSGIGWGIYA GEIDA WGQGTLVTVSS | SEQ ID NO: 25 |
| | EVQLLESGGGLVQPGGSLRLSCAASGFTFSRHGMYWV RQAPGKGLEWVSGIGSSGTGTYYGSAVRGRFTISRDN SKNTLYLQMNSLRAEDTAVYYCARDYGSDSGVGWGIYA GEIDA WGQGTLVTVSS | SEQ ID NO: 26 |
| | AVTLDESGGGLQTPGGGLSLVCKASGFSFSDYGMGWV RQAPGKGLEFVASISNTGSYTDYGAAVKGRATISRDNG QSTLRLQLNNLRAEDTGTYFCAKSAYGGSYGSSSIDAW GHGTEVIVSS | SEQ ID NO: 27 |
| | AVTLDESGGGLQTPGGGLSLVCKASGFAFGRSSMGWV RQAPGKGLEFVASISNTGSYTDYGAAVKGRATISRDNG QSTLRLQLNNLRAEDTGTYFCAKSAYGGSYGSSSIDA WGHGTEVIVSS | SEQ ID NO: 28 |
| | AVTLDESGGGLQTPGGGLSLVCKASGFSFSDYGMGWV RQAPGKGLEFVAFLGSSSSYTDYGAAVKGRATISRDNG QSTLRLQLNNLRAEDTGTYFCAKSAYGGSYGSSSIDA WGHGTEVIVSS | SEQ ID NO: 29 |

### Example 2. Expression and Purification of Anti-CD22 Recombinant Antibody Proteins

### 2.1 Anti-CD22 scFv-Cκ-HA fusion protein

A gene encoding anti-CD22 scFv was subcloned into a modified pCEP4 mammalian expression vector for the expression of a protein comprising a human constant kappa region (Cκ) and an HA (Hemagglutinin) tag (Experimental & Molecular Medicine (2014) 46, e114; doi:10.1038/emm.2014.57). The resulting construct was transfected into HEK293F cells, and the scFv-Cκ-HA fusion protein was purified using KappaSelect Resin (GE Healthcare) according to the manufacturer's instructions.

Examples of the anti-CD22 scFv-Cκ-HA fusion proteins obtained by this method are shown in Table 5.

**[Table 5]**

| | | Sequence | SEQ ID |
|---|---|---|---|
| Recom binant anti-CD22 scFv-Cκ fusion protein | 2-3-14 | LTQPSSVSANPGETVKITCSGSNNTYGWYQQ KAPGSAPVTVIYDNTNRPSDIPSRFSGSKSGS TNTLTITGVQAEDEAVYFCGSRDSTYVGIFGA GTTLTVL GGGGSGGGGSGGGGS AVTLDESGGGLQTPGGGLSLVCKASGFSFSD YGMGWVRQAPGKGLEFVASISNTGSYTDYG AAVKGRATISRDNGQSTLRLQLNNLRAEDTGT YFCAKSAYGGSYGSSSIDAWGHGTEVIVSS GGGGSGGGGSGGGGS RTVAAPSVFIFPPSDEQLKSGTASVVCLLNNF YPREAKVQWKVDNALQSGNSQESVTEQDSK DSTYSLSSTLTLSKADYEKHKVYACEVTHQGL SSPVTKSFNRGESGGAYPYDVPDYAS | SEQ ID NO: 30 |
| | C14-47 | LTQPSSVSANPGETVKITCSGGSGGYGWYQ QKSPGSAPVSLIYDNDQRPSDIPSRFSGSKS GSTGTLTITGVQADDEAVYYCGSYDSSYVGIF GAGTTLTVL GGGGSGGGGSGGGGS AVTLDESGGGLQTPGGALSLVCKASGFTFSN YCMQWVRQAPGKGLEWVAGIGSSGTGTYYG SAVRGRATISRDNGQSTVRLQLNNLRAEDTG TYYCARDYGSDSGCGWGIYAGEIDAWGHGT EVIVSS GGGGSGGGGSGGGGS RTVAAPSVFIFPPSDEQLKSGTASVVCLLNNF YPREAKVQWKVDNALQSGNSQESVTEQDSK DSTYSLSSTLTLSKADYEKHKVYACEVTHQGL SSPVTKSFNRGESGGAYPYDVPDYAS | SEQ ID NO: 31 |

### 2.2 Anti-CD22 human IgG protein

To express the anti-CD22 human IgG protein, genes encoding the human immunoglobulin heavy and light chain regions were subcloned into a mammalian expression vector. Subsequently, the heavy and light chain constructs were combined and transfected into HEK293F cells, and the human IgG protein was purified using MabSelect Resin (GE Healthcare) according to the manufacturer's instructions.

Examples of the anti-CD22 human IgG proteins obtained by this method are shown in Table 6.

**[Table 6]**

| | | Sequence | SEQ ID |
|---|---|---|---|
| Reco mbin ant anti-CD2 2 IgG protei n | 2-3-14 | >light chain | SEQ ID NO: 32 |
| | | LTQPSSVSANPGETVKITCSGSNNTYGWYQQK APGSAPVTVIYDNTNRPSDIPSRFSGSKSGSTN TLTITGVQAEDEAVYFCGSRDSTYVGIFGAGTTL TVL RTVAAPSVFIFPPSDEQLKSGTASVVCLLNNFYP REAKVQWKVDNALQSGNSQESVTEQDSKDST YSLSSTLTLSKADYEKHKVYACEVTHQGLSSPV TKSFNRGEC | |
| | | >heavy chain | SEQ ID NO: 33 |
| | | AVTLDESGGGLQTPGGGLSLVCKASGFSFSDY GMGWVRQAPGKGLEFVASISNTGSYTDYGAAV KGRATISRDNGQSTLRLQLNNLRAEDTGTYFCA KSAYGGSYGSSSIDAWGHGTEVIVSS ASTKGPSVFPLAPCSRSTSESTAALGCLVKDYF PEPVTVSWNSGALTSGVHTFPAVLQSSGLYSLS SVVTVPSSSLGTKTYTCNVDHKPSNTKVDKRV ESKYGPPCPPCPAPEFLGGPSVFLFPPKPKDTL MISRTPEVTCVVVDVSQEDPEVQFNWYVDGVE VHNAKTKPREEQFNSTYRVVSVLTVLHQDWLN GKEYKCKVSNKGLPSSIEKTISKAKGQPREPQV YTLPPSQEEMTKNQVSLTCLVKGFYPSDIAVEW ESNGQPENNYKTTPPVLDSDGSFFLYSRLTVDK SRWQEGNVFSCSVMHEALHNHYTQKSLSLSL GK | |
| | 2-3-14_H1 -24 | >light chain | SEQ ID NO: 34 |
| | | LTQPSSVSANPGETVKITCSGSNNTYGWYQQK APGSAPVTVIYDNTNRPSDIPSRFSGSKSGSTN TLTITGVQAEDEAVYFCGSRDSTYVGIFGAGTTL TVL RTVAAPSVFIFPPSDEQLKSGTASVVCLLNNFYP REAKVQWKVDNALQSGNSQESVTEQDSKDST YSLSSTLTLSKADYEKHKVYACEVTHQGLSSPV TKSFNRGEC | |
| | | >heavy chain | SEQ ID NO: 35 |
| | | AVTLDESGGGLQTPGGGLSLVCKASGFAFGRS SMGWVRQAPGKGLEFVASISNTGSYTDYGAAV KGRATISRDNGQSTLRLQLNNLRAEDTGTYFCA KSAYGGSYGSSSIDAWGHGTEVIVSS ASTKGPSVFPLAPCSRSTSESTAALGCLVKDYF PEPVTVSWNSGALTSGVHTFPAVLQSSGLYSLS SVVTVPSSSLGTKTYTCNVDHKPSNTKVDKRV ESKYGPPCPPCPAPEFLGGPSVFLFPPKPKDTL MISRTPEVTCVVVDVSQEDPEVQFNWYVDGVE VHNAKTKPREEQFNSTYRVVSVLTVLHQDWLN GKEYKCKVSNKGLPSSIEKTISKAKGQPREPQV YTLPPSQEEMTKNQVSLTCLVKGFYPSDIAVEW ESNGQPENNYKTTPPVLDSDGSFFLYSRLTVDK SRWQEGNVFSCSVMHEALHNHYTQKSLSLSL GK | |
| | 2-3-14_H2 -40 | >light chain | SEQ ID NO: 36 |
| | | LTQPSSVSANPGETVKITCSGSNNTYGWYQQK APGSAPVTVIYDNTNRPSDIPSRFSGSKSGSTN TLTITGVQAEDEAVYFCGSRDSTYVGIFGAGTTL TVL RTVAAPSVFIFPPSDEQLKSGTASVVCLLNNFYP REAKVQWKVDNALQSGNSQESVTEQDSKDST YSLSSTLTLSKADYEKHKVYACEVTHQGLSSPV TKSFNRGEC | |
| | | | |
| | | >heavy chain | SEQ ID NO: 37 |
| | | AVTLDESGGGLQTPGGGLSLVCKASGFSFSDY GMGWVRQAPGKGLEFVAFLGSSSSYTDYGAA VKGRATISRDNGQSTLRLQLNNLRAEDTGTYFC AKSAYGGSYGSSSIDAWGHGTEVIVSS ASTKGPSVFPLAPCSRSTSESTAALGCLVKDYF PEPVTVSWNSGALTSGVHTFPAVLQSSGLYSLS SVVTVPSSSLGTKTYTCNVDHKPSNTKVDKRV ESKYGPPCPPCPAPEFLGGPSVFLFPPKPKDTL MISRTPEVTCVVVDVSQEDPEVQFNWYVDGVE VHNAKTKPREEQFNSTYRVVSVLTVLHQDWLN GKEYKCKVSNKGLPSSIEKTISKAKGQPREPQV YTLPPSQEEMTKNQVSLTCLVKGFYPSDIAVEW ESNGQPENNYKTTPPVLDSDGSFFLYSRLTVDK SRWQEGNVFSCSVMHEALHNHYTQKSLSLSL GK | |
| | CI4-47_8-1 | >light chain | SEQ ID NO: 38 |
| | | LTQPPSVSVSPGQTARITCSGGSGGYGWYQQ KPGSAPVSVIYDNDQRPSGIPERFSGSSSGTTV TLTISGVQAEDEADYYCGSYDSSYVGIFGGGTK LTVL RTVAAPSVFIFPPSDEQLKSGTASVVCLLNNFYP REAKVQWKVDNALQSGNSQESVTEQDSKDST YSLSSTLTLSKADYEKHKVYACEVTHQGLSSPV TKSFNRGEC | |
| | | >heavy chain | SEQ ID NO: 39 |
| | | EVQLLESGGGLVQPGGSLRLSCAASGFTFSNY CMSWVRQAPGKGLEWVSGIGSSGTGTYYGSA VRGRFTISRDNSKNTLYLQMNSLRAEDTAVYYC ARDYGSDSGCGWGIYAGEIDAWGQGTLVTVSS | |
| | | ASTKGPSVFPLAPCSRSTSESTAALGCLVKDYF PEPVTVSWNSGALTSGVHTFPAVLQSSGLYSLS SVVTVPSSSLGTKTYTCNVDHKPSNTKVDKRV ESKYGPPCPPCPAPEFLGGPSVFLFPPKPKDTL MISRTPEVTCVVVDVSQEDPEVQFNWYVDGVE VHNAKTKPREEQFNSTYRVVSVLTVLHQDWLN GKEYKCKVSNKGLPSSIEKTISKAKGQPREPQV YTLPPSQEEMTKNQVSLTCLVKGFYPSDIAVEW ESNGQPENNYKTTPPVLDSDGSFFLYSRLTVDK SRWQEGNVFSCSVMHEALHNHYTQKSLSLSL GK | |
| | CI4-47_8-1_H1-2-1 | >light chain | SEQ ID NO: 40 |
| | | LTQPPSVSVSPGQTARITCSGGSGGYGWYQQ KPGSAPVSVIYDNDQRPSGIPERFSGSSSGTTV TLTISGVQAEDEADYYCGSYDSSYVGIFGGGTK LTVL RTVAAPSVFIFPPSDEQLKSGTASVVCLLNNFYP REAKVQWKVDNALQSGNSQESVTEQDSKDST YSLSSTLTLSKADYEKHKVYACEVTHQGLSSPV TKSFNRGEC | |
| | | >heavy chain | SEQ ID NO: 41 |
| | | EVQLLESGGGLVQPGGSLRLSCAASGFTFSKH GHYWVRQAPGKGLEWVSGIGSSGTGTYYGSA VRGRFTISRDNSKNTLYLQMNSLRAEDTAVYYC ARDYGSDSGIGWGIYAGEIDAWGQGTLVTVSS ASTKGPSVFPLAPCSRSTSESTAALGCLVKDYF PEPVTVSWNSGALTSGVHTFPAVLQSSGLYSLS SVVTVPSSSLGTKTYTCNVDHKPSNTKVDKRV ESKYGPPCPPCPAPEFLGGPSVFLFPPKPKDTL MISRTPEVTCVVVDVSQEDPEVQFNWYVDGVE VHNAKTKPREEQFNSTYRVVSVLTVLHQDWLN | |
| | | GKEYKCKVSNKGLPSSIEKTISKAKGQPREPQV YTLPPSQEEMTKNQVSLTCLVKGFYPSDIAVEW ESNGQPENNYKTTPPVLDSDGSFFLYSRLTVDK SRWQEGNVFSCSVMHEALHNHYTQKSLSLSL GK | |
| | CI4-47_8-1_H1-2-37 | >light chain | SEQ ID NO: 42 |
| | | LTQPPSVSVSPGQTARITCSGGSGGYGWYQQ KPGSAPVSVIYDNDQRPSGIPERFSGSSSGTTV TLTISGVQAEDEADYYCGSYDSSYVGIFGGGTK LTVL RTVAAPSVFIFPPSDEQLKSGTASVVCLLNNFYP REAKVQWKVDNALQSGNSQESVTEQDSKDST YSLSSTLTLSKADYEKHKVYACEVTHQGLSSPV TKSFNRGEC | |
| | | >heavy chain | SEQ ID NO: 43 |
| | | EVQLLESGGGLVQPGGSLRLSCAASGFTFSRH GMYWVRQAPGKGLEWVSGIGSSGTGTYYGSA VRGRFTISRDNSKNTLYLQMNSLRAEDTAVYYC ARDYGSDSGVGWGIYAGEIDAWGQGTLVTVSS ASTKGPSVFPLAPCSRSTSESTAALGCLVKDYF PEPVTVSWNSGALTSGVHTFPAVLQSSGLYSLS SVVTVPSSSLGTKTYTCNVDHKPSNTKVDKRV ESKYGPPCPPCPAPEFLGGPSVFLFPPKPKDTL MISRTPEVTCVVVDVSQEDPEVQFNWYVDGVE VHNAKTKPREEQFNSTYRVVSVLTVLHQDWLN GKEYKCKVSNKGLPSSIEKTISKAKGQPREPQV YTLPPSQEEMTKNQVSLTCLVKGFYPSDIAVEW ESNGQPENNYKTTPPVLDSDGSFFLYSRLTVDK SRWQEGNVFSCSVMHEALHNHYTQKSLSLSL GK | |

### 2.4 Anti-CD22 x Anti-cotinine Bispecific Antibody Protein

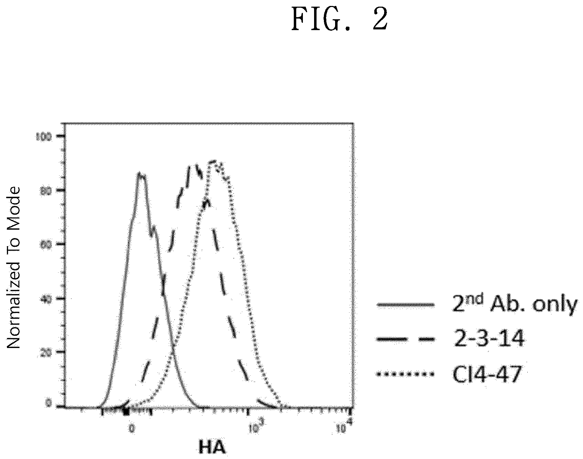

To express the anti-CD22 x anti-cotinine bispecific antibody protein, genes encoding an anti-CD22 scFv, a human constant kappa region (Cκ), and an anti-cotinine scFv (US20080226650A1, Fig 2) were subcloned into a modified pCEP4 mammalian expression vector. The resulting construct was transfected into HEK293F cells, and the scFv-Cκ-scFv fusion protein was purified using KappaSelect Resin (GE Healthcare) according to the manufacturer's instructions.

Examples of the anti-CD22 x anti-cotinine bispecific antibody proteins obtained by this method are shown in Table 7.

**[Table 7]**

| | | Sequence | SEQ ID |
|---|---|---|---|
| Anti-CD22 x Anti-cotinin e Bispec ific Antibo dy Protei n | 2-3-14_ Cκ_anti-cotinine | LTQPSSVSANPGETVKITCSGSNNTYGWYQQ KAPGSAPVTVIYDNTNRPSDIPSRFSGSKSGS TNTLTITGVQAEDEAVYFCGSRDSTYVGIFGA GTTLTVL GGGGSGGGGSGGGGS AVTLDESGGGLQTPGGGLSLVCKASGFSFSD YGMGWVRQAPGKGLEFVASISNTGSYTDYG AAVKGRATISRDNGQSTLRLQLNNLRAEDTGT YFCAKSAYGGSYGSSSIDAWGHGTEVIVSS GGGGSGGGGSGGGGS RTVAAPSVFIFPPSDEQLKSGTASVVCLLNNF YPREAKVQWKVDNALQSGNSQESVTEQDSK DSTYSLSSTLTLSKADYEKHKVYACEVTHQGL SLPVTKSFNRGES GGGGSGGGGSGGGGS ELDLTQTPASVSAAVGGTVTINCQSSQSPYSN EWLSWYQQKPGQAPKVLISRISTLASGVSSR FKGSGSGTQFTLTISDLECGDAATYFCAGGY NFGLFPFGGGTELEIL SSGGGGSGGGGGGSSRSS QSVKESEGRLVTPGGSLTLTCTVSGIDLSRDW | SEQ ID NO: 44 |
| | | MNWVRQAPGEGLEWIGAIGRSGDTYYATWA KGRFTISKTSSRTVTLTVTDLQRSDTATYFCA RIPYFGWNNGDIWGPGTLVTI | |
| | CI4-47_ Cκ_anti-cotinine | LTQPSSVSANPGETVKITCSGGSGGYGWYQ QKSPGSAPVSLIYDNDQRPSDIPSRFSGSKS GSTGTLTITGVQADDEAVYYCGSYDSSYVGIF GAGTTLTVL GGGGSGGGGSGGGGS AVTLDESGGGLQTPGGALSLVCKASGFTFSN YCMQWVRQAPGKGLEWVAGIGSSGTGTYYG SAVRGRATISRDNGQSTVRLQLNNLRAEDTG TYYCARDYGSDSGCGWGIYAGEIDAWGHGT EVIVSS GGGGSGGGGSGGGGS RTVAAPSVFIFPPSDEQLKSGTASVVCLLNNF YPREAKVQWKVDNALQSGNSQESVTEQDSK DSTYSLSSTLTLSKADYEKHKVYACEVTHQGL SLPVTKSFNRGES GGGGSGGGGSGGGGS ELDLTQTPASVSAAVGGTVTINCQSSQSPYSN EWLSWYQQKPGQAPKVLISRISTLASGVSSR FKGSGSGTQFTLTISDLECGDAATYFCAGGY NFGLFPFGGGTELEIL SSGGGGSGGGGGGSSRSS QSVKESEGRLVTPGGSLTLTCTVSGIDLSRDW MNWVRQAPGEGLEWIGAIGRSGDTYYATWA KGRFTISKTSSRTVTLTVTDLQRSDTATYFCA RIPYFGWNNGDIWGPGTLVTI | SEQ ID NO: 45 |
| | Hercepti n_ Cκ_anti-cotinine | DIQMTQSPSSLSASVGDRVTITCRASQDVNTA VAWYQQKPGKAPKLLIYSASFLYSGVPSRFS GSRSGTDFTLTISSLQPEDFATYYCQQHYTTP | SEQ ID NO: 46 |
| | | PTFGQGTKVEIK GGSSRSSSSGGGGSGGGG EVQLVESGGGLVQPGGSLRLSCAASGFNIKD TYIHWVRQAPGKGLEWVARIYPTNGYTRYAD SVKGRFTISADTSKNTAYLQMNSLRAEDTAVY YCSRWGGDGFYAMDYWGQGTLVTVSS GGGGSGGGGSGGGGS RTVAAPSVFIFPPSDEQLKSGTASVVCLLNNF YPREAKVQWKVDNALQSGNSQESVTEQDSK DSTYSLSSTLTLSKADYEKHKVYACEVTHQGL SLPVTKSFNRGES GGGGSGGGGSGGGGS ELDLTQTPASVSAAVGGTVTINCQSSQSPYSN EWLSWYQQKPGQAPKVLISRISTLASGVSSR FKGSGSGTQFTLTISDLECGDAATYFCAGGY NFGLFPFGGGTELEIL SSGGGGSGGGGGGSSRSS QSVKESEGRLVTPGGSLTLTCTVSGIDLSRDW MNWVRQAPGEGLEWIGAIGRSGDTYYATWA KGRFTISKTSSRTVTLTVTDLQRSDTATYFCA RIPYFGWNNGDIWGPGTLVTI | |

### 2.4 Anti-CD22 x Anti-CD3 Bispecific Antibody Protein

To express the anti-CD22 x anti-CD3 bispecific antibody protein, genes encoding an anti-CD22 scFv, a GGGGS peptide linker, an anti-CD3 scFv derived from blinatumomab, and a hexahistidine tag were subcloned into a modified pCEP4 mammalian expression vector. The resulting construct was transfected into HEK293F cells, and the scFv-GGGGS-scFv-His₆ fusion protein was purified using Anti-His Resin (GenScript, USA) according to the manufacturer's instructions.

Examples of the anti-CD22 x anti-CD3 bispecific antibody proteins obtained by this method are shown in Table 8.

**[Table 8]**

| | | Sequence | SEQ ID |
|---|---|---|---|
| Anti-CD22 x anti-CD3 bispecifi c antibod y | CI4-47 x anti-CD3( blinat umom ab) | LTQPSSVSANPGETVKITCSGGSGGYGWYQQKSP GSAPVSLIYDNDQRPSDIPSRFSGSKSGSTGTLTIT GVQADDEAVYYCGSYDSSYVGIFGAGTTLTVL GGGGSGGGGSGGGGS AVTLDESGGGLQTPGGALSLVCKASGFTFSNYCMQ WVRQAPGKGLEWVAGIGSSGTGTYYGSAVRGRATI SRDNGQSTVRLQLNNLRAEDTGTYYCARDYGSDS GCGWGIYAGEIDAWGHGTEVIVSS GGGGS DIKLQQSGAELARPGASVKMSCKTSGYTFTRYTMH WVKQRPGQGLEWIGYINPSRGYTNYNQKFKDKATL TTDKSSSTAYMQLSSLTSEDSAVYYCARYYDDHYC LDYWGQGTTLTVSSVE GGSGGSGGSGGS GGVDDIQLTQSPAIMSASPGEKVTMTCRASSSVSY MNWYQQKSGTSPKRWIYDTSKVASGVPYRFSGSG SGTSYSLTISSMEAEDAATYYCQQWSSNPLTFGAG TKLELK HHHHHH | SEQ ID NO: 47 |
| | CI4-47_8-1 x anti-CD3( blinat umom ab) | LTQPPSVSVSPGQTARITCSGGSGGYGWYQQKPG SAPVSVIYDNDQRPSGIPERFSGSSSGTTVTLTISG VQAEDEADYYCGSYDSSYVGIFGGGTKLTVL GGGGSGGGGSGGGGS EVQLLESGGGLVQPGGSLRLSCAASGFTFSNYCMS WVRQAPGKGLEWVSGIGSSGTGTYYGSAVRGRFT ISRDNSKNTLYLQMNSLRAEDTAVYYCARDYGSDS GCGWGIYAGEIDAWGQGTLVTVSS GGGGS DIKLQQSGAELARPGASVKMSCKTSGYTFTRYTMH WVKQRPGQGLEWIGYINPSRGYTNYNQKFKDKATL TTDKSSSTAYMQLSSLTSEDSAVYYCARYYDDHYC LDYWGQGTTLTVSSVE GGSGGSGGSGGS GGVDDIQLTQSPAIMSASPGEKVTMTCRASSSVSY MNWYQQKSGTSPKRWIYDTSKVASGVPYRFSGSG SGTSYSLTISSMEAEDAATYYCQQWSSNPLTFGAG TKLELK HHHHHH | SEQ ID NO: 48 |
| | CI4-47_8-1_H1-2-1 x anti-CD3( blinat umom ab) | LTQPPSVSVSPGQTARITCSGGSGGYGWYQQKPG SAPVSVIYDNDQRPSGIPERFSGSSSGTTVTLTISG VQAEDEADYYCGSYDSSYVGIFGGGTKLTVLGGGG SGGGGSGGGGS EVQLLESGGGLVQPGGSLRLSCAASGFTFSKHGHY WVRQAPGKGLEWVSGIGSSGTGTYYGSAVRGRFT ISRDNSKNTLYLQMNSLRAEDTAVYYCARDYGSDS GIGWGIYAGEIDAWGQGTLVTVSS GGGGS DIKLQQSGAELARPGASVKMSCKTSGYTFTRYTMH WVKQRPGQGLEWIGYINPSRGYTNYNQKFKDKATL TTDKSSSTAYMQLSSLTSEDSAVYYCARYYDDHYC LDYWGQGTTLTVSSVE GGSGGSGGSGGS GGVDDIQLTQSPAIMSASPGEKVTMTCRASSSVSY MNWYQQKSGTSPKRWIYDTSKVASGVPYRFSGSG SGTSYSLTISSMEAEDAATYYCQQWSSNPLTFGAG TKLELK HHHHHH | SEQ ID NO: 49 |
| | CI4-47_8-1_H1-2-37 x anti-CD3( blinat umom ab) | LTQPPSVSVSPGQTARITCSGGSGGYGWYQQKPG SAPVSVIYDNDQRPSGIPERFSGSSSGTTVTLTISG VQAEDEADYYCGSYDSSYVGIFGGGTKLTVLGGGG SGGGGSGGGGS EVQLLESGGGLVQPGGSLRLSCAASGFTFSRHGM YWVRQAPGKGLEWVSGIGSSGTGTYYGSAVRGRF TISRDNSKNTLYLQMNSLRAEDTAVYYCARDYGSD SGVGWGIYAGEIDAWGQGTLVTVSS GGGGS DIKLQQSGAELARPGASVKMSCKTSGYTFTRYTMH WVKQRPGQGLEWIGYINPSRGYTNYNQKFKDKATL TTDKSSSTAYMQLSSLTSEDSAVYYCARYYDDHYC LDYWGQGTTLTVSSVE GGSGGSGGSGGS GGVDDIQLTQSPAIMSASPGEKVTMTCRASSSVSY MNWYQQKSGTSPKRWIYDTSKVASGVPYRFSGSG SGTSYSLTISSMEAEDAATYYCQQWSSNPLTFGAG TKLELK HHHHHH | SEQ ID NO: 50 |
| | 2-3-14 x anti-CD3( blinat umom ab) | LTQPSSVSANPGETVKITCSGSNNTYGWYQQKAPG SAPVTVIYDNTNRPSDIPSRFSGSKSGSTNTLTITGV QAEDEAVYFCGSRDSTYVGIFGAGTTLTVLGGGGS GGGGSGGGGS AVTLDESGGGLQTPGGGLSLVCKASGFSFSDYGM GWVRQAPGKGLEFVASISNTGSYTDYGAAVKGRAT ISRDNGQSTLRLQLNNLRAEDTGTYFCAKSAYGGS YGSSSIDAWGHGTEVIVSS GGGGS DIKLQQSGAELARPGASVKMSCKTSGYTFTRYTMH WVKQRPGQGLEWIGYINPSRGYTNYNQKFKDKATL TTDKSSSTAYMQLSSLTSEDSAVYYCARYYDDHYC LDYWGQGTTLTVSSVE GGSGGSGGSGGS GGVDDIQLTQSPAIMSASPGEKVTMTCRASSSVSY MNWYQQKSGTSPKRWIYDTSKVASGVPYRFSGSG SGTSYSLTISSMEAEDAATYYCQQWSSNPLTFGAG TKLELK HHHHHH | SEQ ID NO: 51 |
| | 2-3-14_H 1-24 x anti-CD3( blinat umom ab) | LTQPSSVSANPGETVKITCSGSNNTYGWYQQKAPG SAPVTVIYDNTNRPSDIPSRFSGSKSGSTNTLTITGV QAEDEAVYFCGSRDSTYVGIFGAGTTLTVLGGGGS GGGGSGGGGS AVTLDESGGGLQTPGGGLSLVCKASGFAFGRSSM GWVRQAPGKGLEFVASISNTGSYTDYGAAVKGRAT ISRDNGQSTLRLQLNNLRAEDTGTYFCAKSAYGGS YGSSSIDAWGHGTEVIVSS GGGGS DIKLQQSGAELARPGASVKMSCKTSGYTFTRYTMH WVKQRPGQGLEWIGYINPSRGYTNYNQKFKDKATL TTDKSSSTAYMQLSSLTSEDSAVYYCARYYDDHYC LDYWGQGTTLTVSSVE GGSGGSGGSGGS GGVDDIQLTQSPAIMSASPGEKVTMTCRASSSVSY MNWYQQKSGTSPKRWIYDTSKVASGVPYRFSGSG SGTSYSLTISSMEAEDAATYYCQQWSSNPLTFGAG TKLELK HHHHHH | SEQ ID NO: 52 |
| | 2-3-14_H 2-40 x anti-CD3( blinat umom ab) | LTQPSSVSANPGETVKITCSGSNNTYGWYQQKAPG SAPVTVIYDNTNRPSDIPSRFSGSKSGSTNTLTITGV QAEDEAVYFCGSRDSTYVGIFGAGTTLTVLGGGGS GGGGSGGGGS AVTLDESGGGLQTPGGGLSLVCKASGFSFSDYGM GWVRQAPGKGLEFVAFLGSSSSYTDYGAAVKGRA TISRDNGQSTLRLQLNNLRAEDTGTYFCAKSAYGG SYGSSSIDAWGHGTEVIVSS GGGGS DIKLQQSGAELARPGASVKMSCKTSGYTFTRYTMH WVKQRPGQGLEWIGYINPSRGYTNYNQKFKDKATL TTDKSSSTAYMQLSSLTSEDSAVYYCARYYDDHYC LDYWGQGTTLTVSSVE GGSGGSGGSGGS | SEQ ID NO: 53 |
| Blinatumomab | | DIQLTQSPASLAVSLGQRATISCKASQSVDYDGDSY LNWYQQIPGQPPKLLIYDASNLVSGIPPRFSGSGSG TDFTLNIHPVEKVDAATYHCQQSTEDPWTFGGGTK LEIK GGGGSGGGGSGGGGS QVQLQQSGAELVRPGSSVKISCKASGYAFSSYWM NWVKQRPGQGLEWIGQIWPGDGDTNYNGKFKGK ATLTADESSSTAYMQLSSLASEDSAVYFCARRETTT VGRYYYAMDYWGQGTTVTVSS GGGGS DIKLQQSGAELARPGASVKMSCKTSGYTFTRYTMH WVKQRPGQGLEWIGYINPSRGYTNYNQKFKDKATL TTDKSSSTAYMQLSSLTSEDSAVYYCARYYDDHYC LDYWGQGTTLTVSSVE GGSGGSGGSGGS GGVDDIQLTQSPAIMSASPGEKVTMTCRASSSVSY MNWYQQKSGTSPKRWIYDTSKVASGVPYRFSGSG SGTSYSLTISSMEAEDAATYYCQQWSSNPLTFGAG TKLELK HHHHHH | SEQ ID NO: 54 |

### Example 3. Binding Analysis using Anti-CD22 Recombinant Antibody Proteins 3.1 ELISA Analysis using Anti-CD22 scFv-Cκ-HA Fusion Protein

Microtiter plates (Corning Inc., MA, USA) were coated overnight at 4 °C with human CD22-His protein (100 ng/well, Sino Biological Inc., Beijing, China). The wells were blocked for 1 hour at 37 °C with 150 µl of 3 % BSA (w/v) in PBS, and serial dilutions of the anti-CD22 scFv-Cκ-HA fusion protein, prepared in 3% BSA in PBS, were added to each well. After incubation for 1.5 hours at 37 °C, the plates were washed three times with PBST (PBS containing 0.05 % Tween-20), followed by incubation for 1 hour at 37 °C with an HRP-conjugated anti-human Cκ antibody (Millipore, CA, USA). The plates were washed three times with PBST, and the amount of bound antibody was determined by adding 2,2'-azino-bis(3-ethylbenzothiazoline-6-sulfonic acid) (Thermo Fisher Scientific Inc., MA, USA). The resulting OD₄₀₅ was measured using a microplate spectrophotometer.

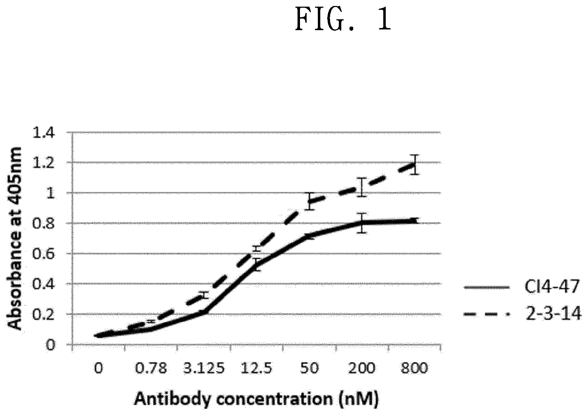

The results of the enzyme immunoassay performed on the recombinant anti-CD22 scFv-Cκ-HA fusion protein are shown in FIG. 1.

FIG. 1 is a graph showing the results of the enzyme immunoassay for the recombinant anti-CD22 scFv-Cκ-HA fusion protein.

As shown in FIG. 1, the recombinant anti-CD22 scFv-Cκ-HA fusion proteins (CI4-47, 2-3-14) were confirmed to bind well to CD22.

### 3.2 Flow Cytometry Analysis using Anti-CD22 scFv-Cκ-HA Fusion Protein

OCI-Ly7 cells were aliquoted into 1.5 ml tubes at a density of 3 x 10⁵ cells per tube. The anti-CD22 scFv-Cκ-HA fusion protein (0.25 µM) was added to each tube, followed by incubation on ice for 30 minutes. After washing twice with MACS buffer (Miltenyi Biotec), the cells were incubated with Alexa Fluor 647-labeled mouse anti-HA antibody (Clone #912426, R&D Systems, MN, USA) on ice for 30 minutes. Then, the cells were washed twice with MACS buffer, resuspended in 150 µL of 1 % paraformaldehyde in MACS buffer, and stored overnight at 4 °C. The cells were analyzed by flow cytometry using a FACS Canto II instrument (BD Bioscience, San Jose, CA, USA). For each sample, 10,000 cells were analyzed.

The results of the flow cytometry analysis performed on the recombinant anti-CD22 scFv-Cκ-HA fusion protein are shown in FIG. 2.

FIG. 2 is a graph showing the results of flow cytometry analysis for anti-CD22 scFv-Cκ-HA fusion proteins 2-3-14 and CI4-47.

As shown in FIG. 2, the recombinant anti-CD22 scFv-Cκ-HA fusion proteins (CI4-47, 2-3-14) demonstrated strong binding to OCI-Ly7 cells.

### 3.3 ELISA Analysis using Anti-CD22 human IgG

Microtiter plates (Corning Inc., MA, USA) were coated overnight at 4 °C with human CD22-His protein (100 ng/well, Sino Biological Inc., Beijing, China). The wells were blocked for 1 hour at 37 °C with 150 µl of 3% BSA (w/v) in PBS, after which serial dilutions of the IgG protein prepared in 3% BSA in PBS were added to each well. After incubation for 1.5 hours at 37 °C, the plates were washed three times with PBST (PBS containing 0.05% Tween-20), followed by incubation for 1 hour at 37 °C with an HRP-conjugated anti-human IgG (Fc-specific) antibody (Thermo Fisher Scientific, USA). The plates were washed three times with PBST, and the amount of bound antibody was determined by adding 2,2'-azino-bis(3-ethylbenzothiazoline-6-sulfonic acid) (Thermo Fisher Scientific Inc., MA, USA). The resulting OD₄₀₅ was measured using a microplate spectrophotometer.

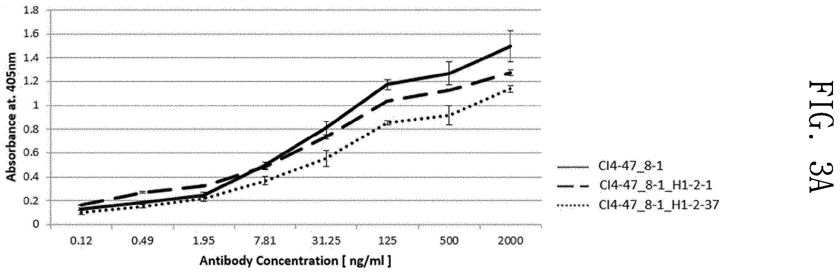

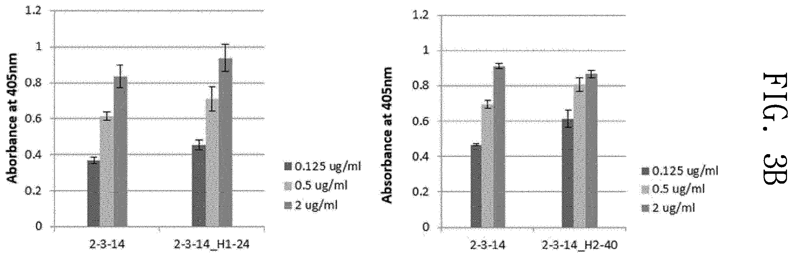

The results of the enzyme immunoassay performed for the recombinant anti-CD22 proteins are shown in FIG. 3.

FIG. 3 shows graphs depicting the enzyme immunoassay results for recombinant anti-CD22 IgG proteins:

FIG. 3A shows results for anti-CD22 IgG proteins CI4-47_8-1, CI4-47_8-1_H1-2-1, and CI4-47_8-1_H1-2-37; FIG. 3B shows results for anti-CD22 IgG proteins 2-3-14, 2-3-14_H1-24, and 2-3-14_H2-40.

As shown in FIG. 3, the anti-CD22 IgG proteins exhibited strong binding to CD22.

### 3.4 Flow Cytometry Analysis Using Anti-CD22 Human IgG

Raji cells were aliquoted into 1.5 ml tubes at a density of 3 x 10⁵ cells per tube. The anti-CD22 IgG protein (0.1 µM) was added to each tube, followed by incubation on ice for 30 minutes. After washing twice with MACS buffer (Miltenyi Biotec), the cells were incubated on ice for 30 minutes with an APC-labeled mouse anti-human constant kappa (Cκ) region antibody. The cells were then washed twice with MACS buffer, resuspended in 150 µL of 1 % paraformaldehyde in MACS buffer, and stored overnight at 4 °C. The cells were analyzed by flow cytometry using a FACS Canto II instrument. For each sample, 10,000 cells were analyzed.

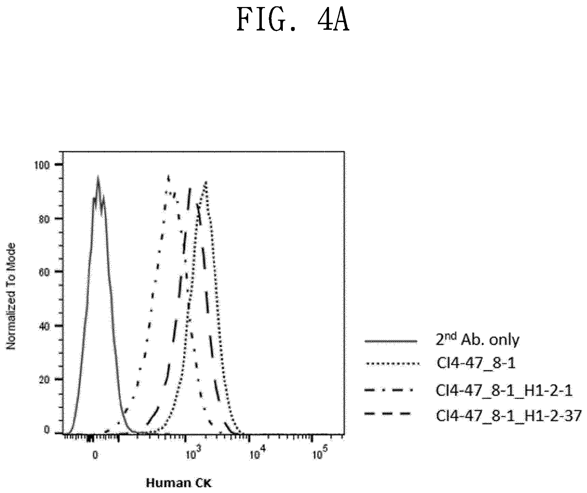

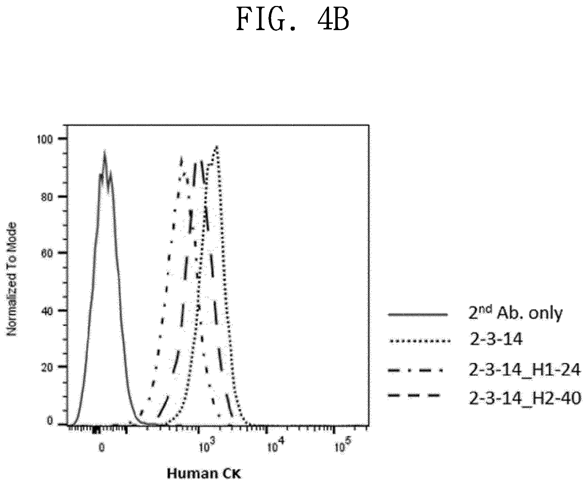

FIG. 4 shows graphs depicting the results of the flow cytometry analysis of recombinant anti-CD22 IgG proteins:

FIG. 4A shows results for anti-CD22 IgG proteins Cl4-47_8-1, CI4-47_8-1_H1-2-1, and CI4-47_8-1_H1-2-37; FIG. 4B shows results for anti-CD22 IgG proteins 2-3-14, 2-3-14_H1-24, and 2-3-14_H2-40.

As shown in FIG. 4, the recombinant anti-CD22 IgG proteins exhibited strong binding to Raji cells.

### Example 4. Construction of Anti-CD22 Single or Anti-CD19/CD22 Bispecific CAR

The CD19 CAR-expression vector (FMC63-CD8 hinge-BBz-pCL20C-MND) was provided by Dr. Byoung Y. Ryu (St. Jude Hospital, Memphis, TN, USA), and the pCAG vector backbone was constructed with reference to pCAGGS of Jun-ichi Miyazaki [25]. This vector includes a CD8 signal peptide, FMC63 scFv, a CD8 hinge and transmembrane domain, a 4-1BB cytoplasmic domain (intracellular co-stimulatory domain), and a CD3ζ signaling domain. The CD19 CAR construct was prepared by tagging a MYC gene to the 5' end of the FMC63 scFv gene within this vector.

The FMC63 scFv gene of the CD19 CAR construct was removed by restriction enzyme digestion, and the C14-47 gene was cloned to prepare a CD22 single CAR construct.

The FMC63 scFv gene of the CD19 CAR construct was removed by restriction enzyme digestion, and genes encoding FMC63 scFv_CI4-47, CI4-47_FMC63 scFv, LOOPCAR6_FMC63 scFv_Cl4-47, and FMC63 scFv_2-3-14 were cloned to prepare CD19/CD22 bispecific CAR constructs.

Examples of the CAR constructs prepared by this method are shown in Table 9.

**[Table 9]**

| | | Sequence | SEQ ID |
|---|---|---|---|
| CD19 CAR | FMC63_CD8 | MALPVTALLLPLALLLHAARPEQKLISEEDL DIQMTQTTSSLSASLGDRVTISCRASQDIS KYLNWYQQKPDGTVKLLIYHTSRLHSGVP | SEQ ID NO: 55 |
| | | SRFSGSGSGTDYSLTISNLEQEDIATYFCQ QGNTLPYTFGGGTKLEITGGGGSGGGGS GGGGSEVKLQESGPGLVAPSQSLSVTCTV SGVSLPDYGVSWIRQPPRKGLEWLGVIW GSETTYYNSALKSRLTIIKDNSKSQVFLKM NSLQTDDTAIYYCAKHYYYGGSYAMDYW GQGTSVTVSSTTTPAPRPPTPAPTIASQPL SLRPEACRPAAGGAVHTRGLDFACDIYIWA PLAGTCGVLLLSLVITLYCKRGRKKLLYIFK QPFMRPVQTTQEEDGCSCRFPEEEEGGC ELRVKFSRSADAPAYKQGQNQLYNELNLG RREEYDVLDKRRGRDPEMGGKPRRKNPQ EGLYNELQKDKMAEAYSEIGMKGERRRGK GHDGLYQGLSTATKDTYDALHMQALPPR | |
| CD22CA R | CI4-47_CD8 | MALPVTALLLPLALLLHAARPEQKLISEEDL LTQPSSVSANPGETVKITCSGGSGGYGWY QQKSPGSAPVSLIYDNDQRPSDIPSRFSG SKSGSTGTLTITGVQADDEAVYYCGSYDS SYVGIFGAGTTLTVLGQSSRSSGGGGSSG GGGSAVTLDESGGGLQTPGGALSLVCKAS GFTFSNYCMQWVRQAPGKGLEWVAGIGS SGTGTYYGSAVRGRATISRDNGQSTVRLQ LNNLRAEDTGTYYCARDYGSDSGCGWGI YAGEIDAWGHGTEVIVSSTTTPAPRPPTPA PTIASQPLSLRPEACRPAAGGAVHTRGLDF ACDIYIWAPLAGTCGVLLLSLVITLYCKRGR KKLLYIFKQPFMRPVQTTQEEDGCSCRFP EEEEGGCELRVKFSRSADAPAYKQGQNQ LYNELNLGRREEYDVLDKRRGRDPEMGG KPRRKNPQEGLYNELQKDKMAEAYSEIGM KGERRRGKGHDGLYQGLSTATKDTYDALH | SEQ ID NO: 56 |
| | | MQALPPR | |
| CD19/ CD22 bispecifi c CAR | FMC63_CI4-47 | MALPVTALLLPLALLLHAARPEQKLISEEDL GRADIQMTQTTSSLSASLGDRVTISCRAS QDISKYLNWYQQKPDGTVKLLIYHTSRLHS GVPSRFSGSGSGTDYSLTISNLEQEDIATY FCQQGNTLPYTFGGGTKLEITGSTSGSGK PGSGEGSTKGEVKLQESGPGLVAPSQSLS VTCTVSGVSLPDYGVSWIRQPPRKGLEWL GVIWGSETTYYNSALKSRLTIIKDNSKSQV FLKMNSLQTDDTAIYYCAKHYYYGGSYAM DYWGQGTSVTVSSGGGGSGGGGSGGG GSLTQPSSVSANPGETVKITCSGGSGGYG WYQQKSPGSAPVSLIYDNDQRPSDIPSRF SGSKSGSTGTLTITGVQADDEAVYYCGSY DSSYVGIFGAGTTLTVLGQSSRSSGGGGS SGGGGSAVTLDESGGGLQTPGGALSLVC KASGFTFSNYCMQWVRQAPGKGLEWVA GIGSSGTGTYYGSAVRGRATISRDNGQST VRLQLNNLRAEDTGTYYCARDYGSDSGC GWGIYAGEIDAWGHGTEVIVSSLINTTTPA PRPPTPAPTIASQPLSLRPEACRPAAGGAV HTRGLDFACDIYIWAPLAGTCGVLLLSLVIT LYCKRGRKKLLYIFKQPFMRPVQTTQEED GCSCRFPEEEEGGCELRVKFSRSADAPAY KQGQNQLYNELNLGRREEYDVLDKRRGR DPEMGGKPRRKNPQEGLYNELQKDKMAE AYSEIGMKGERRRGKGHDGLYQGLSTATK DTYDALHMQALPPR | SEQ ID NO: 57 |
| | CI4-47_ FMC63 | MALPVTALLLPLALLLHAARPEQKLISEEDL GRALTQPSSVSANPGETVKITCSGGSGGY GWYQQKSPGSAPVSLIYDNDQRPSDIPSR | SEQ ID NO: 58 |
| | | FSGSKSGSTGTLTITGVQADDEAVYYCGS YDSSYVGIFGAGTTLTVLGQSSRSSGGGG SSGGGGSAVTLDESGGGLQTPGGALSLV CKASGFTFSNYCMQWVRQAPGKGLEWV AGIGSSGTGTYYGSAVRGRATISRDNGQS TVRLQLNNLRAEDTGTYYCARDYGSDSG CGWGIYAGEIDAWGHGTEVIVSSGGGGS GGGGSGGGGSDIQMTQTTSSLSASLGDR VTISCRASQDISKYLNWYQQKPDGTVKLLI YHTSRLHSGVPSRFSGSGSGTDYSLTISNL EQEDIATYFCQQGNTLPYTFGGGTKLEITG STSGSGKPGSGEGSTKGEVKLQESGPGL VAPSQSLSVTCTVSGVSLPDYGVSWIRQP PRKGLEWLGVIWGSETTYYNSALKSRLTII KDNSKSQVFLKMNSLQTDDTAIYYCAKHY YYGGSYAMDYWGQGTSVTVSSLINTTTPA PRPPTPAPTIASQPLSLRPEACRPAAGGAV HTRGLDFACDIYIWAPLAGTCGVLLLSLVIT LYCKRGRKKLLYIFKQPFMRPVQTTQEED GCSCRFPEEEEGGCELRVKFSRSADAPAY KQGQNQLYNELNLGRREEYDVLDKRRGR DPEMGGKPRRKNPQEGLYNELQKDKMAE AYSEIGMKGERRRGKGHDGLYQGLSTATK DTYDALHMQALPPR | |
| | LOOPCAR6_FM C63_CI4-47 | MALPVTALLLPLALLLHAARPEQKLISEEDL GRADIQMTQTTSSLSASLGDRVTISCRAS QDISKYLNWYQQKPDGTVKLLIYHTSRLHS GVPSRFSGSGSGTDYSLTISNLEQEDIATY FCQQGNTLPYTFGGGTKLEITGGGGSAVT LDESGGGLQTPGGALSLVCKASGFTFSNY CMQWVRQAPGKGLEWVAGIGSSGTGTYY | SEQ ID NO: 59 |
| | | GSAVRGRATISRDNGQSTVRLQLNNLRAE DTGTYYCARDYGSDSGCGWGIYAGEIDA WGHGTEVIVSSGSTSGSGKPGSGEGSTK GLTQPSSVSANPGETVKITCSGGSGGYG WYQQKSPGSAPVSLIYDNDQRPSDIPSRF SGSKSGSTGTLTITGVQADDEAVYYCGSY DSSYVGIFGAGTTLTVLGGGGSEVKLQES GPGLVAPSQSLSVTCTVSGVSLPDYGVSW IRQPPRKGLEWLGVIWGSETTYYNSALKS RLTIIKDNSKSQVFLKMNSLQTDDTAIYYCA KHYYYGGSYAMDYWGQGTSVTVSSLINTT TPAPRPPTPAPTIASQPLSLRPEACRPAAG GAVHTRGLDFACDIYIWAPLAGTCGVLLLS LVITLYCKRGRKKLLYIFKQPFMRPVQTTQE EDGCSCRFPEEEEGGCELRVKFSRSADA PAYKQGQNQLYNELNLGRREEYDVLDKRR GRDPEMGGKPRRKNPQEGLYNELQKDKM AEAYSEIGMKGERRRGKGHDGLYQGLSTA TKDTYDALHMQALPPR | |

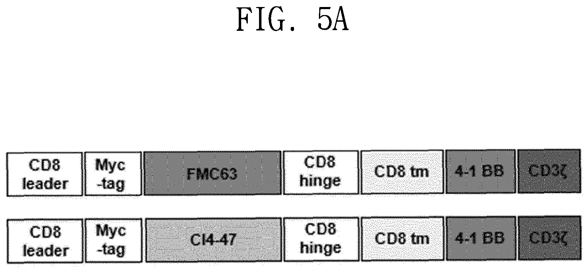

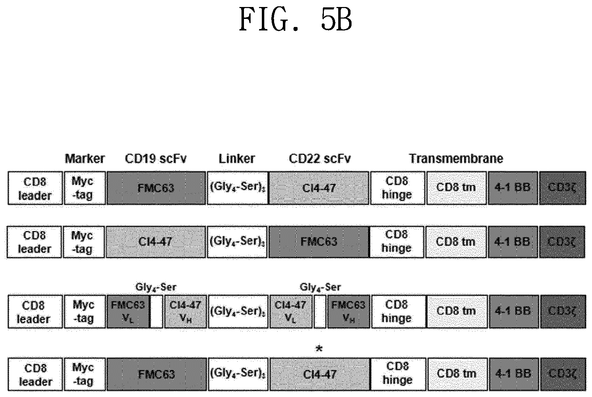

FIG. 5 is a schematic diagram illustrating anti-CD19 and anti-CD22 monospecific and bispecific CAR structures: FIG. 5A is a schematic diagram illustrating anti-CD19 and anti-CD22 monospecific CAR structures. Monospecific CARs were generated by incorporating anti-CD19 (FMC63) and anti-CD22 (CI4-47) antigen-binding domains.

FIG. 5B is a schematic diagram illustrating anti-CD19/CD22 bispecific CAR structures. Anti-CD19/CD22 bispecific CAR structures were generated using four different backbone structures optimized for the CAR structure in NK cells.

### Example 5. In Vitro Evaluation of Anti-CD22 and Anti-CD19/CD22 Bispecific CAR-NK-92 Cells

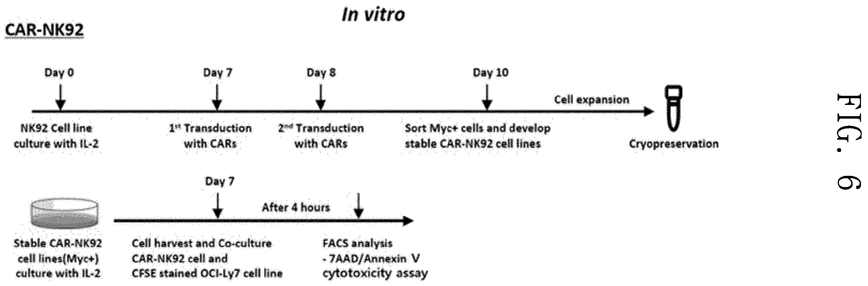

CAR-NK-92 cells were transduced twice with a lentiviral vector expressing a CAR structure, and the resulting Myc+ cells were sorted within 48 hours. The CAR-NK-92 cells were then cryopreserved. For the in vitro evaluation of anti-CD19/CD22 bispecific CAR-NK-92 cells, CAR-NK-92 cells were co-cultured for 4 hours with CFSE-labeled OCI-Ly7 cells, and AV/7-AAD staining was measured by flow cytometry. The results are shown in FIGS. 6 to 8.

FIG. 6 is a schematic diagram illustrating the in vitro evaluation method for anti-CD19, anti-CD22, and anti-CD19/CD22 bispecific CAR-NK-92 cells.

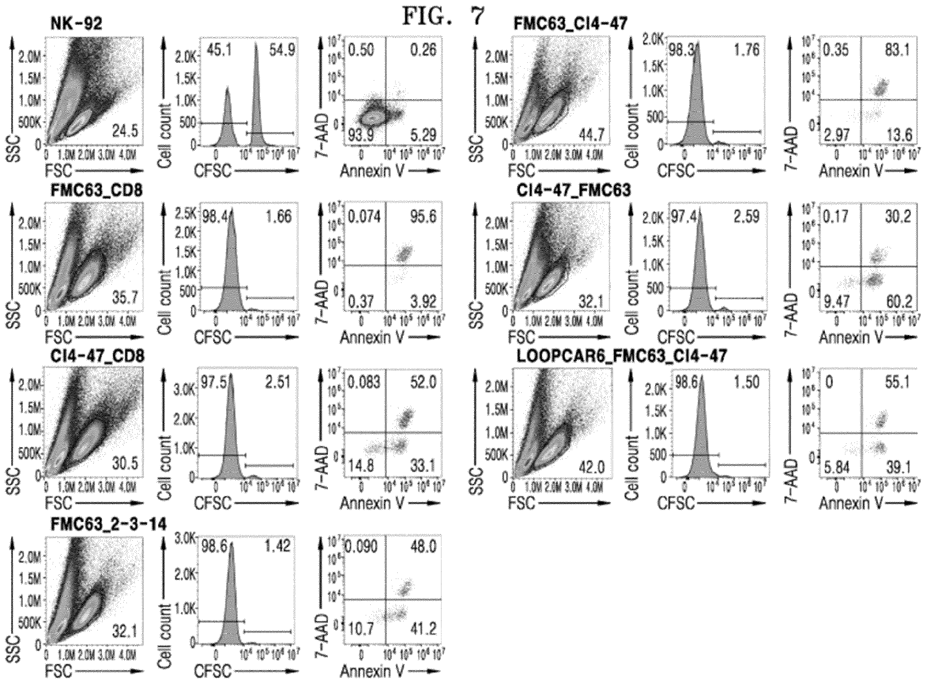

FIG. 7 is a graph showing AV/7-AAD staining measured by flow cytometry following co-culture of CAR-NK-92 cells with CFSE-labeled OCI-Ly7 cells.

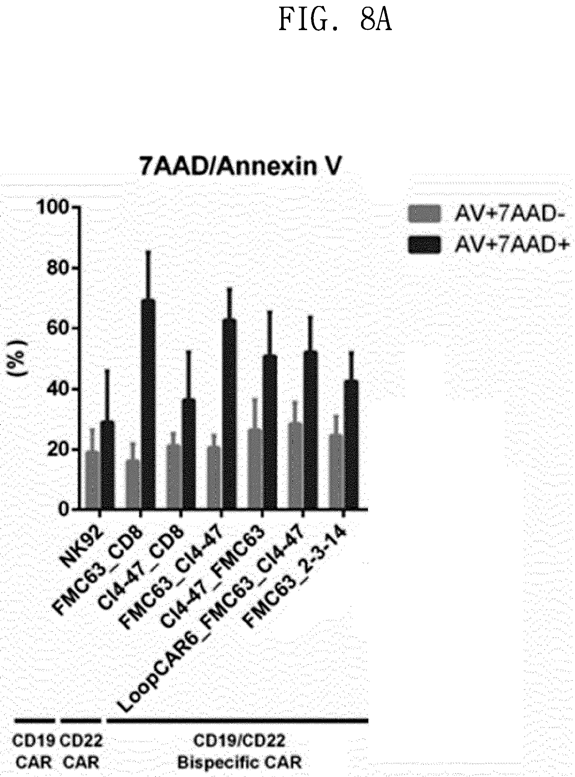

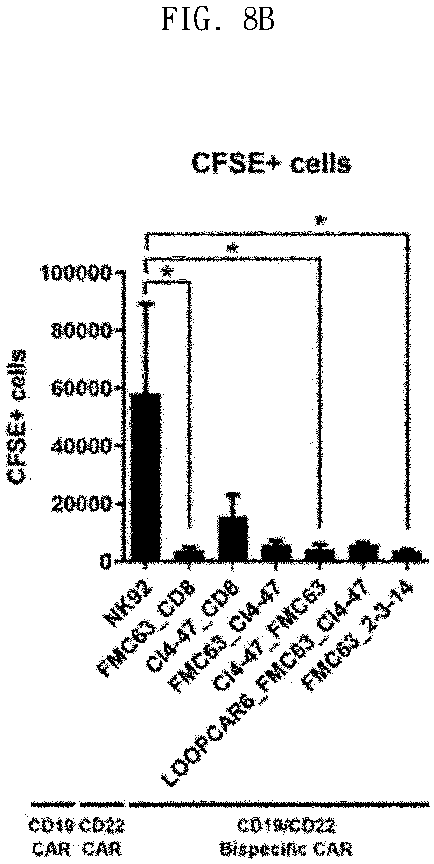

FIG. 8A is a graph showing the percentage of AV+ 7-AAD- early apoptotic cells and AV+ 7-AAD+ late apoptotic cells among CFSE+ target cells, and FIG. 8B is a graph showing the number of CFSE+ target cells.

As shown in FIGS. 6 to 8, increased cancer cell was observed in the groups treated with anti-CD22 or anti-CD19/CD22 bispecific CAR-NK-92 cells compared to the group treated with NK-92 cells alone.

### Example 6. In Vivo Evaluation of Anti-CD22, Anti-CD19/CD22 Bispecific CAR-NK-92 Cells

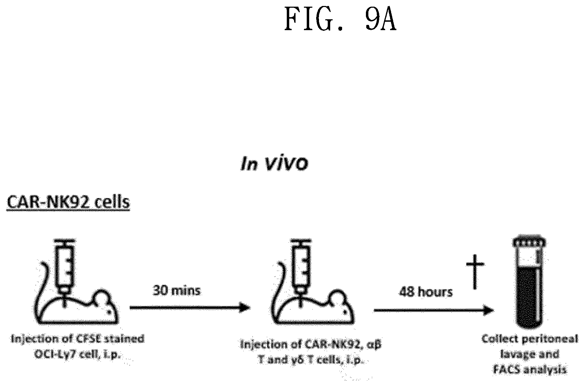

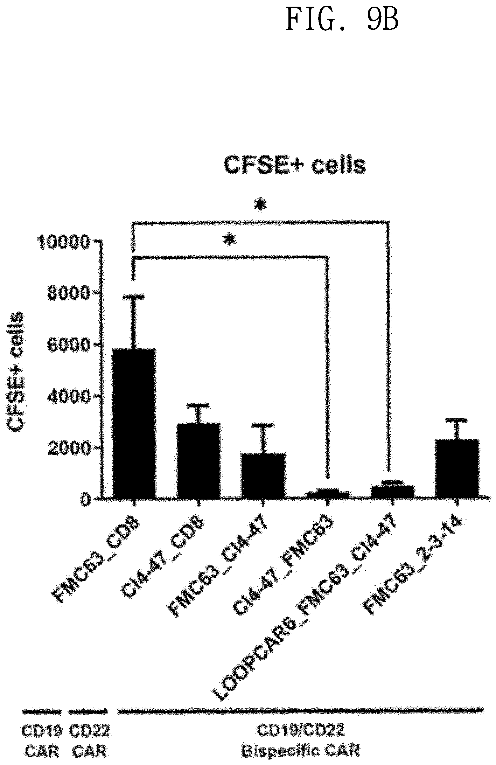

CFSE-labeled OCI-Ly7 cells were injected intraperitoneally (i.p.) into NSG mice, followed 30 minutes later by intraperitoneal injection of CAR-NK-92 cells. After 48 hours, peritoneal lavage fluid was collected, and the number of CFSE⁺ target cells was measured by flow cytometry. The process and results of the in vivo evaluation for anti-CD22 and anti-CD19/CD22 bispecific CAR-NK-92 cells described in Example 5 are shown in FIG. 9.

FIG. 9 shows results illustrating the in vivo cancer cell-killing ability of anti-CD22 and anti-CD19/CD22 bispecific CAR-NK-92 cells:

FIG. 9A is a schematic diagram illustrating the in vivo experimental method, and FIG. 9B is a graph showing the in vivo cancer cell-killing ability of anti-CD22 and anti-CD19/CD22 bispecific CAR-NK-92 cells.

As shown in FIG. 9, anti-CD22 and anti-CD19/CD22 bispecific CAR-NK-92 cells eliminated cancer cells in vivo.

### Example 7. In Vitro Cytotoxicity Test of Anti-CD22 Antibody-Drug Conjugates

### 7.1 In Vitro Cytotoxicity Test of Anti-CD22 x Anti-cotinine Bispecific Antibody & Cotinine-Duocarmycin

A complex including an anti-CD22 x anti-cotinine bispecific antibody and cotinine-duocarmycin was prepared by mixing the antibody protein prepared in Example 2.3 and duocarmycin-conjugated cotinine (conjugated by Levena Biopharma, USA) at equimolar concentrations, followed by incubation for 30 minutes at room temperature.

Raji cells, a B-cell lymphoma cell line, were treated at various concentrations with the following substances, respectively, to assess cytotoxicity: a complex including anti-CD22 (CI4-47, 2-3-14) x anti-cotinine bispecific antibody and cotinine-duocarmycin; a complex including Herceptin x anti-cotinine bispecific antibody and cotinine-duocarmycin (negative control); drug only (cotinine-duocarmycin); and DMSO.

Specifically, to test the in vitro cytotoxicity of the anti-CD22 x anti-cotinine bispecific antibody & cotinine-duocarmycin, Raji cells were seeded into microtiter plates one day prior at a density of 4.5 × 10^3 cells/50 µl per well. After preparing the antibody-drug complex as described above, the complex was diluted with RPMI1640 cell culture medium, and 50 µl of the diluted complex was added to each well containing Raji cells. This antibody-drug complex was diluted with RPMI1640 cell culture medium, and 50 µl of the diluted complex was added to each well containing Raji cells. After 72 hours, luminescence from viable cells was measured using CellTiter-Glo (Promega, USA) according to the manufacturer's instructions.

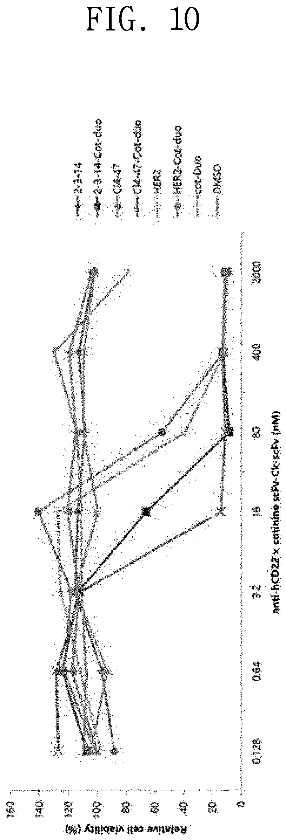

The results of the in vitro cytotoxicity assay for the anti-CD22 x anti-cotinine bispecific antibody & cotinine-duocarmycin are shown in FIG. 10.

FIG. 10 is a graph showing the in vitro cytotoxicity results for the anti-CD22 x anti-cotinine bispecific antibody & cotinine-duocarmycin complex.

As shown in FIG. 10, increased cancer cell death was observed in the anti-CD22 antibody-treated group compared to the negative control group.

### 7.2 In Vitro Cytotoxicity Test of Anti-CD22 IgG and MMAE_Protein A Complex

An anti-CD22 IgG and MMAE_Protein A complex was prepared by mixing the antibody protein produced in Example 2.2 and MMAE-Protein A (purchased from Levena Biopharma, USA) at equimolar concentrations, followed by incubation at room temperature for 30 minutes.

To assess cytotoxicity, Raji cells, a B-cell lymphoma cell line, were treated at various concentrations with complexes prepared by mixing anti-CD22 IgG proteins (C14-47_8-1, 2-3-14, 2-3-14_H1-24, and 2-3-14_H2-40) and MMAE_Protein A (Levena Biopharma) at equimolar concentrations.

Specifically, to test the in vitro cytotoxicity of the anti-CD22 IgG and MMAE_Protein A complex, Raji cells were seeded in microtiter plates one day prior at a density of 4.5 × 10³ cells/50 µl per well. The anti-CD22 IgG protein and MMAE-Protein A (Levena Biopharma, USA) were mixed at equimolar concentrations and incubated for 30 minutes at room temperature. This antibody-drug complex was diluted in RPMI1640 cell culture medium, and 50 µl of the diluted complex was added to each well containing Raji cells. After 72 hours, the luminescence of viable cells was measured using CellTiter-Glo (Promega, USA) according to the manufacturer's instructions.

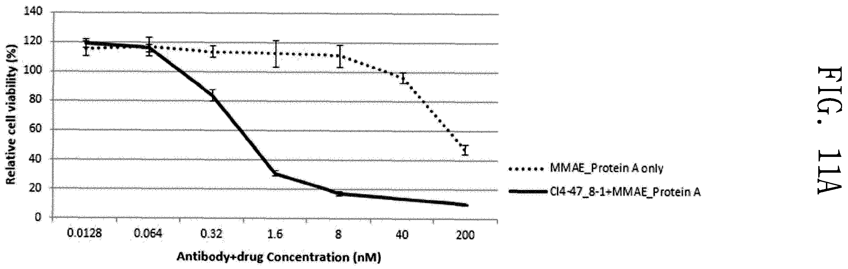

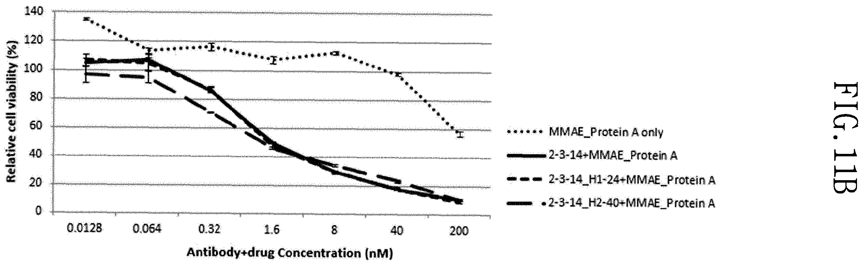

FIG. 11 shows graphs illustrating the in vitro cytotoxicity results of the anti-CD22 IgG and MMAE_Protein A complexes:

FIG. 11A shows the cytotoxicity test results for the complex of anti-CD22 (C14-47_8-1) IgG and MMAE_Protein A, and FIG. 11B is a graph showing the cytotoxicity test results for the complexes of anti-CD22 (2-3-14, 2-3-14_H1-24, and 2-3-14_H2-40) IgG and MMAE_Protein A.

As shown in FIG. 11, increased cancer cell death was observed in the anti-CD22 antibody-treated group compared to the MMAE_Protein A-only treated group.

### Example 8. In Vitro IL-2 Secretion Test of Anti-CD22 x Anti-CD3 Bispecific Antibody

Raji cells, a B-cell lymphoma cell line, were treated with an anti-CD22 (Cl4-47) x anti-CD3 (CD3 scFv from blinatumomab) bispecific antibody or an anti-CD22 (Cl4-47) x irrelevant scFv bispecific antibody as a negative control, at various concentrations. The treated Raji cells were then co-cultured with Jurkat E6.1 cells, a T-cell line, and IL-2 concentration in the cell culture supernatant was measured by ELISA.

Specifically, 1 × 10⁴ Raji cells and 1 × 10⁵ Jurkat E6.1 cells were seeded per well in 96-well U-bottom plates in 150 µl of RPMI1640 cell culture medium. Then, the anti-CD22 x anti-CD3 bispecific antibody was serially diluted in RPMI1640 medium, and 50 µl of the diluted antibody was added to each well. After 72 hours, cell culture supernatants were collected by centrifugation, and the IL-2 concentration was measured using a Human IL-2 ELISA kit (Thermo Fisher Scientific, USA) according to the manufacturer's instructions.

FIG. 12 shows graphs illustrating the in vitro IL-2 secretion assay results for the anti-CD22 x anti-CD3 bispecific antibody:

FIG. 12A shows IL-2 secretion assay results for the C14-47 x anti-CD3 (blinatumomab) bispecific antibody-treated group and the C14-47 x irrelevant antibody negative control antibody-treated group; FIG. 12B shows IL-2 secretion assay results for groups treated with blinatumomab, CI4-47_8-1 x anti-CD3 (blinatumomab), CI4-47_8-1_H1-2-1 x anti-CD3 (blinatumomab), and C14-47_8-1_H1-2-37 x anti-CD3 (blinatumomab) bispecific antibodies; and FIG. 12C shows IL-2 secretion assay results for groups treated with blinatumomab, 2-3-14 x anti-CD3 (blinatumomab), 2-3-14_H1-24 x anti-CD3 (blinatumomab), and 2-3-14_H2-40 x anti-CD3 (blinatumomab) bispecific antibodies.

As shown in FIG. 12, increased IL-2 production was observed in the anti-CD22 x anti-CD3 antibody-treated group compared to the negative control group.

## Claims

1. An antibody or fragment thereof that specifically binds to CD22, comprising:
a light chain variable region comprising a CDR1 region represented by SEQ ID NO: 1 or 2, a CDR2 region represented by SEQ ID NO: 3 or 4, and a CDR3 region represented by SEQ ID NO: 5 or 6; and
a heavy chain variable region comprising a CDR1 region represented by any one of SEQ ID NOS: 7 to 12, a CDR2 region represented by any one of SEQ ID NOS: 13 to 15, and a CDR3 region represented by any one of SEQ ID NOS: 16 to 19.

2. The antibody or fragment thereof of claim 1,
wherein the antibody or antigen-binding fragment thereof comprises: a light chain variable region represented by an amino acid sequence selected from the group consisting of SEQ ID NOS: 20 to 22, and a heavy chain variable region represented by an amino acid sequence selected from the group consisting of SEQ ID NOS: 23 to 29.

3. The antibody or fragment thereof of claim 1,
wherein the antibody or antigen-binding fragment thereof is humanized.

4. The antibody or fragment thereof of claim 3,
wherein the antibody or antigen-binding fragment thereof further comprises: a human constant kappa (C_{K}) region and a hemagglutinin (HA) tag protein; or a human immunoglobulin heavy chain region and a human immunoglobulin light chain region.

5. The antibody or fragment thereof of claim 3,
wherein the antibody or antigen-binding fragment thereof is represented by an amino acid sequence of one of SEQ ID NOS: 30 and 43.

6. An anti-CD22 and anti-cotinine bispecific antibody comprising:
an anti-CD22 antibody or fragment thereof; and
an anti-cotinine antibody or fragment thereof;
wherein the anti-CD22 antibody or fragment thereof is as defined in any one of claims 1 to 5.

7. The anti-CD22 and anti-cotinine bispecific antibody of claim 6, wherein the anti-CD22 and anti-cotinine bispecific antibody is represented by an amino acid sequence of SEQ ID NO: 44 or 46.

8. An anti-CD22 and anti-CD3 bispecific antibody comprising:
an anti-CD22 antibody or fragment thereof; and
an anti-CD3 antibody or fragment thereof;
wherein the anti-CD22 antibody or fragment thereof is as defined in any one of claims 1 to 5.

9. The anti-CD22 and anti-CD3 bispecific antibody of claim 8, wherein the anti-CD22 and anti-CD3 bispecific antibody or fragment thereof comprises an amino acid sequence selected from the group consisting of SEQ ID NOS: 47 to 54.

10. A polynucleotide encoding the antibody or fragment of any one of claims 1 to 9.

11. A vector comprising the polynucleotide of claim 10.

12. A recombinant cell transformed with the vector of claim 11.

13. A chimeric antigen receptor (CAR) comprising:
an antigen-binding domain;
a transmembrane domain;
a hinge domain;
an intracellular co-stimulatory domain; and
an intracellular signal transduction domain,
wherein the antigen-binding domain comprises an antibody or fragment thereof that specifically binds to CD22.

14. The chimeric antigen receptor of claim 13,
wherein the antigen-binding domain comprises: a light chain variable region comprising a CDR1 region represented by SEQ ID NO: 1 or 2, a CDR2 region represented by SEQ ID NO: 3 or 4, and a CDR3 region represented by SEQ ID NO: 5 or 6; and
a heavy chain variable region comprising a CDR1 region represented by an amino acid sequence selected from SEQ ID NOS: 7 to 12, a CDR2 region represented by an amino acid sequence selected from SEQ ID NOS: 13 to 15, and a CDR3 region represented by an amino acid sequence selected from SEQ ID NOS: 16 to 19.

15. The chimeric antigen receptor of claim 13,
wherein the antigen-binding domain comprises: a light chain variable region represented by an amino acid sequence selected from the group consisting of SEQ ID NOS: 20 to 22; and
a heavy chain variable region represented by an amino acid sequence selected from the group consisting of SEQ ID NOS: 23 to 29.

16. The chimeric antigen receptor of claim 13,
wherein the antigen-binding domain is connected to the transmembrane domain via the hinge domain.

17. The chimeric antigen receptor of claim 13,
wherein the intracellular co-stimulatory domain and the intracellular signal transduction domain are sequentially linked to a C-terminal portion of the transmembrane domain.

18. The chimeric antigen receptor of claim 13,
wherein the chimeric antigen receptor is represented by an amino acid sequence of SEQ ID NO: 56.

19. The chimeric antigen receptor of claim 13, targeting CD19 and CD22, wherein the antigen-binding domain further comprises a CD19-binding domain, thereby forming a bispecific chimeric antigen receptor targeting CD19 and CD22.

20. The chimeric antigen receptor of claim 19, targeting CD19 and CD22, wherein the CD19-binding domain is linked via a linker to an antigen-binding domain that specifically binds to CD22.

21. The chimeric antigen receptor of claim 19, targeting CD19 and CD22, wherein the bispecific chimeric antigen receptor comprises a linker connecting a C-terminus of the CD19-binding domain to an N-terminus of the CD22-binding domain.

22. The chimeric antigen receptor of claim 19, targeting CD19 and CD22, wherein the bispecific chimeric antigen receptor comprises a linker connecting a C-terminus of the CD22-binding domain to an N-terminus of the CD19-binding domain.

23. The chimeric antigen receptor of claim 19, targeting CD19 and CD22, wherein the bispecific chimeric antigen receptor comprises, in sequence: a light chain variable region of an antibody that specifically binds to CD19; a heavy chain variable region of an antibody that specifically binds to CD22; a light chain variable region of an antibody that specifically binds to CD22; and a heavy chain variable region of an antibody that specifically binds to CD19.

24. The chimeric antigen receptor of claim 19, wherein the chimeric antigen receptor comprises an amino acid sequence selected from the group consisting of SEQ ID NOS: 57 to 59.

25. A polynucleotide encoding the chimeric antigen receptor of any one of claims 13 to 24.

26. A vector comprising the polynucleotide of claim 25.

27. A recombinant cell transformed with the vector of claim 26.

28. The cell of claim 27,
wherein the cell is any one of NK cells, T cells, cytotoxic T cells, and regulatory T cells.

29. An antibody-drug conjugate comprising:
an anti-CD22 antibody or an antigen-binding fragment thereof, or a bispecific antibody comprising the anti-CD22 antibody or antigen-binding fragment thereof, or an antigen-binding fragment of the bispecific antibody; a drug moiety; and a linker connecting the drug moiety to the antibody or antigen-binding fragment thereof.

30. The antibody-drug conjugate of claim 29 or a pharmaceutically acceptable salt thereof, wherein the linker is a cleavable linker or a non-cleavable linker.

31. A pharmaceutical composition for preventing or treating a B cell-mediated disease, comprising:
the antibody or fragment thereof of claim 1 that specifically binds to CD22;
the anti-CD22 and anti-cotinine bispecific antibody of claim 6;
the anti-CD22 and anti-CD3 bispecific antibody of claim 11;
the chimeric antigen receptor of claim 13 that specifically binds to CD22;
the bispecific chimeric antigen receptor of claim 18 targeting CD19 and CD22; or
the antibody-drug conjugate of claim 28.

32. The pharmaceutical composition of claim 31,
wherein the B cell-mediated disease is selected from the group consisting of tumors, lymphoma, non-Hodgkin's lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma.
